(19) **Europäisches Patentamt / European Patent Office / Office européen des brevets**

(11) **EP 2 393 834 B1**

(12) **EUROPEAN PATENT SPECIFICATION**

(45) Date of publication and mention
of the grant of the patent:
**17.04.2013 Bulletin 2013/16**

(21) Application number: **10705118.7**

(22) Date of filing: **05.02.2010**

(51) Int Cl.:
***C07K 14/79*** (2006.01)

(86) International application number:
**PCT/EP2010/051453**

(87) International publication number:
**WO 2010/089385 (12.08.2010 Gazette 2010/32)**

(54) **PURIFICATION PROCESS**

AUFREINIGUNGSVERFAHREN

PROCÉDÉ DE PURIFICATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **06.02.2009 EP 09152332**

(43) Date of publication of application:
**14.12.2011 Bulletin 2011/50**

(73) Proprietor: **Novozymes Biopharma DK A/S 2880 Bagsvaerd (DK)**

(72) Inventors:
- **BLACKWELL, Lee, Edward**
  **Nottingham**
  **Nottinghamshire NG14 5HU (GB)**
- **CAMERON, Jason**
  **Nottingham**
  **Nottinghamshire NG3 6HB (GB)**
- **MORTON, Phillip, Harvey**
  **Nottingham**
  **Nottinghamshire NG2 6FS (GB)**

(74) Representative: **Williams, Rachel Clare Novozymes Biopharma UK Limited Castle Court 59 Castle Boulevard Nottingham NG7 1FD (GB)**

(56) References cited:
WO-A-00/01407        WO-A-97/49414
WO-A-02/080980       WO-A-03/086272
WO-A1-2005/003152    SU-A1- 436 854

- DE CREMER K ET AL: "Behaviour of vanadate and vanadium-transferrin complex on different anion-exchange columns. Application to in vivo <48>V-labelled rat serum" JOURNAL OF CHROMATOGRAPHY B: BIOMEDICAL SCIENCES & APPLICATIONS, ELSEVIER, AMSTERDAM, NL, vol. 775, no. 2, 5 August 2002 (2002-08-05), pages 143-152, XP004369505 ISSN: 1570-0232
- BLIRUP-JENSEN SOREN: "Protein standardization I: Protein purification procedure for the purification of human prealbumin, orosomucoid and transferrin as primary protein preparations" CLINICAL CHEMISTRY AND LABORATORY MEDICINE, WALTER DE GRUYTER & CO, BERLIN, NEW YORK, vol. 39, no. 11, 1 January 2001 (2001-01-01), pages 1076-1089, XP008106690 ISSN: 1434-6621

**Description**

**Reference to sequence listing**

This application contains a Sequence Listing in computer readable form. The computer readable form is incorporated herein by reference.

## FIELD OF THE INVENTION

The invention relates to a process for purifying a transferrin solution. The invention also relates to the product of the purification process. The term 'transferrin' refers to both transferrin and transferrin-like proteins.

## BACKGROUND TO THE INVENTION

The listing or discussion of a prior-published document in this specification should not necessarily be taken as an acknowledgement that the document is part of the state of the art or is common general knowledge.
Transferrin is a protein that is known to regulate the bioavailability of iron to the cell, supporting key metabolic processes such as DNA synthesis and oxygen transport. Human serum transferrin (HST) is the major iron-binding protein in normal human plasma, and is present at about 2-4 g/l (van Campenhout et al, 2003, Free Radic. Res., 37, 1069-1077). Serum transferrin is currently derived from either bovine or human sources and is a common supplement used in commercial scale mammalian cell culture. With increasing pressure from regulatory authorities to reduce animal-derived components in the manufacture of biologics, recombinant transferrin is desirable for the serum-free, large scale mammalian cell culture industry.
More specifically, human plasma transferrin is a monomeric glycoprotein of 678 amino acids with a relative molecular mass of about 80 kDa. Each molecule of transferrin can bind strongly one or two ferric ions.
Purification of transferrin from plasma by chromatography has been described, for example in Rivat et al (1992) Journal of Chromatography, 576: 71-77, US 6,251,860; US 5,744,586; and US 5,041,537. Purification of recombinant transferrin from yeast has been described in PCT/EP2008/060482, and US 5,986,067. Transferrin may also be produced in other hosts, such as in plants
Irrespective of the source of transferrin, be it animal, human or recombinant, it is desirable to recover maximal amounts of transferrin during purification of the transferrin from the source. However, current methods of purification of transferrin do not result in a sufficiently high yield of purified transferrin. Therefore, what is required is an improved purification process for transferrin.

## SUMMARY OF THE INVENTION

The invention provides a chromatographic purification process which results in an increased yield of transferrin. Two steps of the purification process have been found to be particularly beneficial. These two steps may be used independently or together.
Accordingly, the invention provides an improved process for the purification of transferrin from a relatively impure solution of transferrin. The process may include one or both of an anionic chromatography step and a cationic chromatography step.
The invention also provides transferrin purified by the purification method and uses of the transferrin purified by the purification method.

## BRIEF DESCRIPTION OF DRAWINGS

Fig. 1 shows A254nm trace of Capto™Q chromatograph with (A) zero and (B) 824 mol.mole$^{-1}$ (5.4 mM) borate present in the transferrin solution which is loaded onto the chromatography column.
Fig. 2 is a graphical representation, overlay plot, of the SP-FF low pH, high salt wash 2 design space for a host cell protein clearance of at least 275-fold and a recovery of at least 60%.

## DETAILED DESCRIPTION OF THE INVENTION

A first aspect of the invention provides a process for purifying a transferrin solution (the transferrin solution may be considered to be a 'starting material'), the process comprising:

a) adding tetraborate to a relatively impure solution of transferrin to provide a solution of transferrin and tetraborate;

b) applying the solution of transferrin and tetraborate to an anionic chromatographic material for which the transferrin has no specific binding activity such that the transferrin binds to the material;

c) washing the chromatographic material to which the transferrin is bound;

d) optionally further washing the chromatographic material to which the transferrin is bound; and

e) optionally, recovering the transferrin from the anionic chromatographic material.

Preferably the wash of step (c) is carried out using a wash solution having a lower concentration than the wash solution used in the wash of step (d). For example, 'lower' may mean at least 5, 10, 25, 30 or 50% lower and/or by at least 5, 10, 20, 30, 40, or 50 mM or by at least 2-, 3-, 4-, 5- or 10-fold.

More specifically, the process for purifying a transferrin solution may comprise the steps of:

a) adding tetraborate to a relatively impure solution of transferrin to provide a solution of transferrin and tetraborate at a ratio of at least 1 mole tetraborate to 1 mole transferrin, for example at least 20 mole tetraborate to 1 mole transferrin;

b) applying the solution of transferrin and tetraborate to an anionic chromatographic material for which the transferrin has no specific binding affinity such that the transferrin- binds to the material;

c) washing the chromatographic material with a tetraborate solution of from 0.1 mM to 30 mM, for example 0.1 mM to 20 mM;

d) subsequently washing the chromatographic material with a tetraborate solution of from 5 mM to 60 mM, for example 30 to 60 mM or at least 30 mM ; and

e) optionally, recovering the transferrin from the anionic chromatographic material.

The term 'no specific binding activity' means that, preferably, the transferrin does not bind to the chromatographic material in an antibody-antigen binding manner.

A surprising advantage of adding tetraborate to the transferrin solution prior to loading the transferrin on to the anionic chromatography material is that this increases the load capacity of the material for transferrin. The load capacity may be increased by 2-fold, 3-fold or more. Thus more transferrin may be loaded on a given amount of matrix, and/or the amount of transferrin lost in the flow-through may be reduced. Preferably, tetraborate is added at a ratio from 1 mole tetraborate to 1 mole transferrin to 1000 mole tetraborate to 1 mole transferrin, for example at, from or up to at or about 1, 10, 15, 20, 30, 50, 100, 200, 300, 400, 450, 500, 600, 700, 800, 900 or 1000 mol.mol$^{-1}$. A preferred ratio is from 20 to 1000 mole tetraborate to 1 mole transferrin. The advantage of adding tetraborate to transferrin prior to loading the transferrin onto the anionic chromatographic material may be exploited alone or in combination with the wash step(s) of the purification process disclosed above. For example, tetraborate may be used to increase the transferrin-binding capacity of an anionic exchange matrix independently of or in combination with a purification process that uses that matrix.

The tetraborate solution used in the washing of step (c) may be at a concentration of from at or about 0.1 mM to at or about 30 mM. For example, the tetraborate solution used in (c) may be at a concentration from at or about 0.1 mM, 0.5 mM, 1 mM, 2 mM, 4 mM, 6 mM, 8 mM, 10 mM, 15 mM, 20 mM or 25 mM to at or about 0.5 mM, 1 mM, 2 mM, 4 mM, 6 mM, 8 mM, 10 mM, 15 mM, 20 mM, 25 mM or 30 mM. More particularly the tetraborate solution of (c) may be at a concentration from at or about 2.5 mM to at or about 7.5 mM, most particularly at or about 5 mM.

The tetraborate solution used in the washing of step (d) may be at a concentration from at or about 5 mM to at or about 60 mM. For example, the tetraborate solution used in (d) may be at a concentration from at or about 5 mM, 10 mM, 15 mM, 20 mM, 25 mM, 30 mM, 35 mM, 40 mM, 45 mM, 50 mM or 55 mM to at or about 10 mM, 15 mM, 20 mM, 25 mM, 30 mM, 35 mM, 40 mM, 45 mM, 50 mM, 55 mM or 60 mM. More particularly the tetraborate solution of (d) may be at a concentration from at or about 20 mM to at or about 40 mM, most particularly at or about 30 mM.

A surprising advantage of carrying out a low concentration 'pre-wash', such as the wash of step (c), prior to a higher concentration wash, such as the wash of step (d), is that this regime reduces the loss of transferrin during the higher concentration wash. Preferably, 'concentration' is 'tetraborate concentration'.

The tetraborate may be a tetraborate of a Group I metal, such as potassium tetraborate or sodium tetraborate. A preferred tetraborate is potassium tetraborate.

The transferrin may be recovered from the anionic chromatographic material by elution, for example by a change in pH and/or an increase in conductivity. The elution solution may have a pH of less than or equal to pH 5.2 and/or a conductivity of greater than or equal to 2.5 mS.cm$^{-1}$. An alternative elution liquid comprises 110 mM potassium tetraborate or sodium tetraborate.

Preferably the anionic chromatographic material comprises a functional ligand which is a strong anion exchanger or a weak anion exchanger. A functional ligand may be an ion. Examples of strong anion exchangers include quaternary amine groups ("Q"), i.e. -N$^{+}$(CH$_3$)$_3$ such as Q Sepharose Fast Flow (Q-FF) and Capto Q (both available from GE Healthcare, Little Chalfont, UK), UNOsphere™ Q (Bio-Rad, Hemel Hempstead, UK), Toyopeari® SuperQ (TosoH Corporation, Tokyo, Japan), Q Ceramic HyperD® (Pall, Portsmouth, UK) and POROS® 50 HQ (Applied Biosystems Inc., Foster City, CA, USA). Examples of weak anion exchangers include diethylaminoalkyl groups such as diethylaminoethyl groups ("DEAE", also referred to as "DE") i.e. -N⁺H(CH₂CH₃)₂ such as DEAE Sepharose Fast Flow (DE-FF; available from GE Healthcare). Further strong and weak anion exchangers are described in 'Liquid column chromatography: a survey of modern techniques and applications' (1975, pages 344-351, Editors: Zdenek Deyl, Karel Macek, Jaroslav Janák, Publisher: Elsevier) and in 'Protein liquid chromatography' (2000, pages 18-21, Editor: Michael Kastener, Publisher: Elsevier)

Weak anion exchangers may benefit from the use of wash buffers for steps (c) and (d) which are of a lower concentration than those used for strong anion exchangers.

Preferably the transferrin solution is loaded onto the anionic chromatography material at from about 0.01 mg transferrin per mL chromatography matrix to about 100 $mg.mL^{-1}$., for example about from at or about 0.01, 0.1, 1, 10, 20, 30, 40, 50, 60, 70, 80 or 90 $mg.mL^{-1}$ to at or about 0.1, 1, 10, 20, 30, 40, 50, 60, 70, 80, 90 or 100 $mg.mL^{-1}$. Preferred loading parameters are from at or about 10 to at or about 100 $mg.mL^{-1}$. A particularly preferred loading parameter is at or about 30 $mg.mL^{-1}$.

Preferably the transferrin solution which is loaded onto the anionic chromatography matrix has a pH of from about pH 8 to 10, such as from about pH 8.0 to 10.0 more preferably at, to or from pH 9.0 to 9.4.

Prior to loading the transferrin-containing material onto the anionic chromatographic material, the anionic chromatographic material may be equilibrated for example with one or more strengths of tetraborate solution, for example 110 mM potassium tetraborate and/or 30 mM potassium tetraborate. Potassium tetraborate may be partially or fully replaced by a different tetraborate such as a Group I metal tetraborate, such as sodium tetraborate.

A second aspect of the invention provides a process for purifying a transferrin solution (the transferrin solution may be considered to be a 'starting material'), the process comprising an anionic chromatographic step and a cationic chromatographic step, in which:

the cationic chromatographic step comprises:

a) applying a relatively impure solution of a transferrin to a cationic chromatographic material for which the transferrin has no specific affinity such that the transferrin binds to the material;
b) washing the cationic chromatographic material with a wash buffer having a pH of from about 4 to about 5 and being substantially free of salts other than the salt which provides the buffering effect;
c) subsequently washing the cationic chromatographic material with a wash buffer having a pH from about 4 to about 5 and a salt concentration of from about 50 mM to about 5 M; and
d) recovering the transferrin from the cationic chromatographic material; and

the anionic chromatographic step comprises:

a) adding tetraborate to transferrin obtained from step (d) of the cationic chromatographic step to provide a solution of transferrin and tetraborate;
b) applying the solution of transferrin and tetraborate to an anionic chromatographic material for which the transferrin has no specific activity such that the transferrin binds to the material;
c) washing the chromatographic material to which the transferrin is bound;
d) optionally further washing the chromatographic material to which the transferrin is bound; and
e) optionally, recovering the transferrin from the anionic chromatographic material.

Preferably the pH of the wash buffer of step (b) of the cationic chromatographic step is from at or about 4.0, 4.1, 4.2, 4.3, 4.4, 4.5, 4.6, 4.7, 4.8 or 4.9 to at or about 4.1, 4.2, 4.3, 4.4, 4.5, 4.6, 4.7, 4.8, 4.9 or 5.0, most preferably at or about pH 4.5.

Preferably the wash buffer of step (b) of the cationic chromatographic step has a total salt concentration of from about 10 mM to about 200 mM, more preferably at about 50 mM. More preferably the salt which provides the buffering activity of the wash buffer of step (b) of the cationic chromatographic step is present at a concentration of from about 10 mM to about 200 mM, more preferably at about 50 mM. Preferably the wash buffer of step (b) is substantially free of salt, other than the salt providing the buffering effect (i.e. the 'buffering salt') and most preferably is free of salt other than the buffering salt. The term 'substantially free' means that the level of salt, other than buffering salt, is undetectable and or is sufficiently low as to have no effect on the wash buffer relative to a wash buffer that is completely free of salt other than the buffering salt. For example, it is preferred that the wash buffer of step (b) is substantially free of sodium chloride.

It is preferred that the pH of the wash buffer of step (c) of the cationic chromatographic step is at or about 4.0, 4.1, 4.2, 4.3, 4.4, 4.5, 4.6, 4.7, 4.8 or 4.9 to at or about 4.1, 4.2, 4.3, 4.4, 4.5, 4.6, 4.7, 4.8, 4.9 or 5.0, most preferably at or about pH 4.5.

Preferably the wash buffer of step (c) of the cationic chromatographic step has a salt concentration of from about 50 mM to about 5 M, for example from at or about 50 mM, 100 mM, 150 mM, 200 mM, 250 mM, 500 mM, 1 M, 1.25 M, 1.5 M, 1.75 M, 2 M, 2.5 M, 3 M, 3.5 M, 4 M or 4.5 M to at or about 100 mM, 150 mM, 200 mM, 250 mM, 500 mM, 1 M, 1.25 M, 1.5 M, 1.75 M, 2 M, 2.5 M, 3 M, 3.5 M, 4 M, 4.5 M or 5 M, more preferably from at or about 250 mM to at or about 2 M, most preferably at or about 2 M. It is preferred that the wash buffer of step (c) has a higher salt concentration that the wash buffer of step (b). Example of 'higher' include by greater than or equal to 5, 10, 20, 30, 40, 50, 100, 150 mM.

Preferred wash buffers of step (c) of the cationic chromatographic step provide a recovery of at least 60, 70, 80, 90, 95 or 100% and/or a host cell protein (HCP) clearance (e.g. yeast antigen clearance) of at least 200, 225, 250, 275, 300, 325, 350, 375 or 400-fold. Particularly preferred wash buffers provide a recovery of at least 60, 70, 80, 90, 95 or 100% and a HCP (e.g. YA) clearance of at least 200, 225, 250, 275, 300, 325, 350, 375 or 400 and are defined by, that is satisfies, one or more (preferably both) of the following equations:

$$\text{Recovery} = -940.50069 + (441.61018 \times p) + (0.043309 \times s) - (47.57735 \times p^2) - (0.010263 \times p \times s)$$

$$\text{HCP } (\textit{e.g. YA}) \text{ clearance} = 16388.65744 - (10512.30681 \times p) - (1.70101 \times s) + (2257.17601 \times p^2) + (0.78628 \times p \times s) - (159.06573 \times p^3) - (0.090182 \times p^2 \times s)$$

In the above equations, $p$ = pH and $s$ = salt concentration in mM and recovery is % recovery (for example weight/weight).

For example, the wash buffer may provide a recovery of at least 60% and a HCP (e.g. YA) clearance of at least 275-fold. Parameters of a wash buffer providing a recovery of at least 60% and a HCP (e.g. YA) clearance of at least 275-fold may be defined by the following equations:

$$\text{Recovery (\%): } 60 = -940.50069 + (441.61018 \times p) + (0.043309 \times s) - (47.57735 \times p^2) - (0.010263 \times p \times s)$$

$$\text{HCP } (\textit{e.g. YA}) \text{ clearance (fold): } 275 = 16388.65744 - (10512.30681 \times p) - (1.70101 \times s) + (2257.17601 \times p^2) + (0.78628 \times p \times s) - (159.06573 \times p^3) - (0.090182 \times p^2 \times s)$$

In the above equations, $p$ pH and $s$ = salt concentration in mM.

Recovery (%) may be weight/weight. The term 'host cell protein' is well known to the skilled person. Host cell proteins include proteins native to and produced by a host cell which may be present as a contaminant in a protein, such as a heterologous protein, produced by the host cell. It is desirable to reduce, or eliminate, the amount of host cell protein present in a product produced from that host cell. For a yeast host cell, the host cell proteins may be known as 'yeast antigens'.

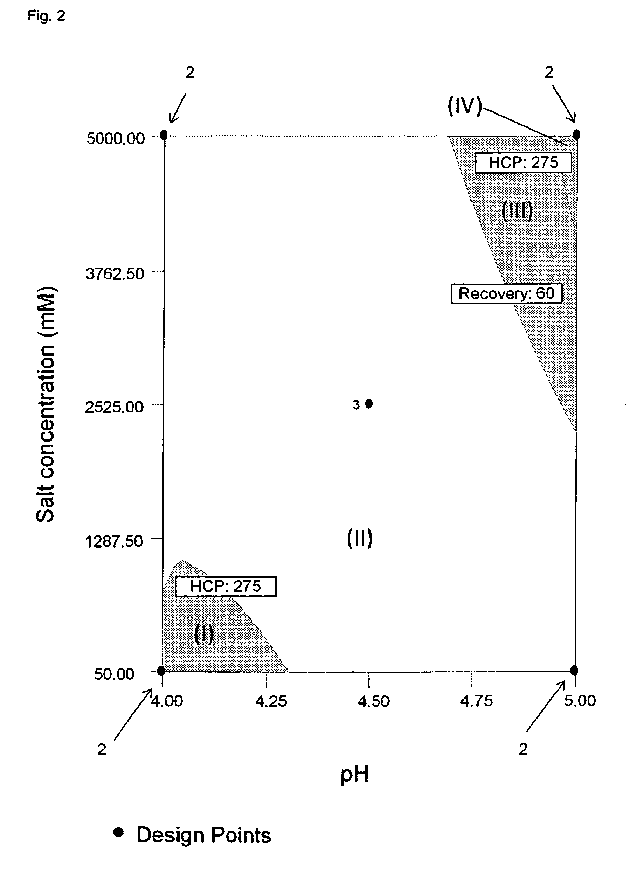

The pH, salt concentration, HCP (e.g. YA) clearance and recovery may be plotted on a graph, e.g. a an overlay plot. Fig.2 shows an example of an overlay plot. Such a graph gives a view of the 'design space'. The design space represents the combinations of pHs and salt concentrations at which the desired recovery (%) and HCP (e.g. YA) clearance (fold) is achieved. A design space can be generated ('defined') for any combination of desired recovery and HCP (YA) clearance.

The design space is the most accurate definition of the combinations of pHs and salt concentrations which provide the desired recovery and HCP (e.g. YA) clearance. However, the combinations may also be approximated by describing at least a part, and preferably a significant part, of the design space by one or more rectangular areas. For example, the design space defined by a recovery of 60% or more and a HCP (e.g. YA) clearance of 275-fold or more may be approximated by one or more of (i) pH 4.3 - 5.0, 50 - 1100mM salt, (ii) pH 4.0 - 4.7, 1100 - 5000mM salt, (iii) pH 4.7 - 5.0, 1100 - 2270mM salt (iv) pH 4.7 - 4.8, 2270 - 3900mM salt, (v) pH 4.8 - 4.9, 2270 - 2990mM salt and (vi) pH 4.7 - 4.75, 3900 - 4345mM salt. Preferably the design is approximated by (i) to (iv), more preferably by (i) to (v) and most preferably by (i) to (vi). Suitable salts include monovalent and divalent metal and ammonium salts. One preferred salt for the cationic chromatographic step is NaCl.

The wash buffer of step (c) may or may not comprise two or more salts. For example, the wash buffer of step (c) may or may not comprise sodium acetate and NaCl. Sodium acetate may be present at or about 27 mM.

A surprising advantage of low pH/high salt wash, such as those described above, is that this improves HCP clearance, e.g. yeast antigen clearance, across the cationic matrix without significantly lowering the recovery of transferrin from the matrix. This is in direct contrast to the expectation that use of such a high salt wash would result in an undesirably high loss of transferrin from the matrix.

The transferrin may be recovered from the cationic chromatographic material by elution, for example with a change of pH and/or conductivity and/or the addition of a specific eluent. The elution solution may comprise sodium phosphate and or sodium chloride. For example sodium phosphate may be present at or about 50 mM. Sodium chloride may be present at or about 150 mM. The pH of the elution solution may be at or about pH 6, for example at or about pH 6.0.

The cationic chromatographic step may comprise a further, for example a third wash, prior to recovery of the transferrin. Preferably the further wash step is carried out after the wash step of step (c). Preferably, the further wash step is carried out immediately after the wash step of step (c), e.g. with no intervening step. The further wash may be carried out using a wash buffer of 50 mM sodium acetate pH 5.0 to 5.2 (preferably pH 5.1) preferably with a conductivity of from 2.5 to 3.1 mS.cm$^{-1}$.

Preferably the transferrin solution is loaded onto the cationic chromatography material at up to 100 mg transferrin per mL chromatographic material, for example from at or about 10, 20, 30, 40, 50, 60, 70, 80 or 90 mg.mL$^{-1}$ to at or about 20, 30, 40, 50, 60, 70, 80, 90 or 100 mg.mL$^{-1}$ more preferably at from about 30 to about 50 mg.mL$^{-1}$.

Preferably the cationic chromatographic material comprises a functional ligand which is a strong cation exchanger. Examples of strong cation exchangers include a sulphonic group, i.e. -SO$_3^-$ e.g. SP Sepharose Fast Flow (SP-FF) and S-Spheradex; (both available from GE Healthcare), SP-Spherosil, SE-Cellulose A particularly preferred cationic chromatographic material is SP Sepharose Fast Flow. The cationic chromatographic material may comprise a functional ligand which is a weak cation exchanger, such materials include, CM-Sepharose FF or CM-Cellulose. Further strong and weak cation exchangers are described in 'Liquid column chromatography: a survey of modem techniques and applications' (1975, pages 344-351, Editors: Zdenek Deyl, Karel Macek, Jaroslav Janák, Publisher: Elsevier) and in 'Protein liquid chromatography' (2000, pages 18-21, Editor: Michael Kastener, Publisher: Elsevier).

Prior to loading (for example, step (a)), the cationic chromatographic material may be equilibrated for example with 50 mM sodium acetate (from at or about pH 4 to pH 6, such as at or about pH 4.0 to pH 6.0, for example from at or about pH 5.0 to at or about pH 5.2, preferably at or about pH 5.1 and with a conductivity of from at or about 2.5 mS.cm$^{-1}$ to at or about 3.1 mS.cm$^{-1}$).

Following recovery of the transferrin, the cationic and/or anionic chromatographic material may be cleaned prior to storage and/or re-use of the material. Techniques for cleaning chromatographic materials are known in the art.

Preferably, the cationic step is followed by the anionic step according to the second aspect of the invention. Alternatively, the cationic step may follow the anionic step. Preferably, the cationic is immediately followed by the anionic step, e.g. with no intervening step. Optionally, other steps may be carried out in between the cationic and anionic steps. The eluate of the cationic step may or may not be diluted prior to being subjected to the anionic step.

Preferably the transferrin solution which is subjected to the purification of the invention is substantially, or completely, free of particles such as cells and cell debris. 'Substantially free' means that any particles present in the solution do not have a significant adverse effect on the purification process. Preferably, any particles present do not reduce the yield of the purification process by more than 10, 9, 8, 7, 6, 5, 4, 3, 2, 1, 0.5 or 0.1 % relative to a solution being completely free of particles.

The final transferrin-containing solution obtained from the process of the first, second or third aspect of the invention may or may not be subjected to one or more further steps such as purification steps. Such further steps may be carried out prior to one or more of the steps of the first, second or third aspects of the invention. For example, the first and second aspects of the invention may be separated by one or more of the further steps.

The one or more further purification steps may be selected from: anion exchange chromatography, cation exchange chromatography, buffer exchange, concentration, dilution, removal of metal ions such as iron ions, dialysis, diafiltration, pH-adjustment, (for example with solution comprising tetraborate and a base, such as potassium tetraborate and sodium hydroxide, particularly 250 mM potassium tetraborate and 0.5 M sodium hydroxide), treatment with a reducing agent, discolouration treatment (e.g. with charcoal) for example to remove yeast-derived and/or matrix-derived colourants, heating (including sterilisation), cooling, conditioning, ultrafiltration, cell separation techniques, such as centrifugation, filtration (e.g. cross-flow filtration, expanded bed chromatography and the like) methods of cell breakage, including beadmilling, sonication, enzymatic exposure and the like, ammonium sulphate precipitation, ethanol precipitation, acid extraction, solvent extraction, phosphocellulose chromatography, hydrophobic interaction chromatography, affinity chromatography, hydroxyapatite chromatography, lectin chromatography, metal affinity/chelating chromatography, high performance liquid chromatography ("HPLC"), reverse phase HPLC, conductivity adjustment, alkaline precipitation of the transferrin to a lipophilic phase and the like. Ultrafiltration is particularly preferred. 'Conditioning' includes adjusting one or more of pH and conductivity and/or the addition of solid and/or ingredients. Ultrafiltration may use a nominal molecular weight cut off of 10 000. Transferrin recovered from the process of the first, second or third aspect of the invention may be pH adjusted to from pH 6.7 to 7.3, for example by the addition of acetic acid. The pH-adjusted transferrin may then be concentrated and/or diafiltered against a NaCl solution. At the end of diafilitration the retentate may be further concentrated.

Any one or more of the above mentioned techniques may or may not be used to further purify the thus isolated protein to a commercially or industrially acceptable level of purity. By commercially or industrially acceptable level of purity, we include the provision of the transferrin protein in which other material (for example, one or more contaminants) are present at a level of less than 50 %, 40 %, 30 %, 20 %, 10 %, 5 %, 4 %, 3 %, 2 %, 1 %, 0.5 %, 0.1 %, 0.01 %, 0.001 %, 0.0001 %, 0.00001 %, or 0.000001 % and, most preferably at a level of 0%.

A commercially or industrially acceptable level of purity may be obtained by a relatively crude purification method by which the protein product of choice is put into a form suitable for its intended purpose. A protein preparation that has been purified to a commercially or industrially acceptable level of purity may, in addition to the protein product of choice, also comprise, for example, cell culture components such as host cells or debris derived therefrom. Alternatively, high molecular weight components (such as host cells or debris derived therefrom) may or may not be removed (such as by filtration or centrifugation) to obtain a composition comprising the protein product of choice and, optionally, a functionally acceptable level of low molecular weight contaminants derived from the cell culture process.

The protein may or may not be purified to achieve a pharmaceutically acceptable level of purity. A protein has a pharmaceutically acceptable level of purity if it is essentially pyrogen free and can be used for its intended purpose and hence be administered in a pharmaceutically efficacious amount without causing medical effects not associated with the activity of the protein.

The transferrin product may be provided at a concentration of at least $10^{-4}$g.L$^{-1}$, $10^{-3}$ g.L$^{-1}$, 0.01 g.L$^{-1}$, 0.02 g.L$^{-1}$, 0.03 g.L$^{-1}$, 0.04 g.L$^{-1}$, 0.05 g.L$^{-1}$, 0.06 g.L$^{-1}$, 0.07 g.L$^{-1}$, 0.08 g.L$^{-1}$, 0.09 g.L$^{-1}$, 0.1 g.L$^{-1}$, 0.2 g.L$^{-1}$, 0.3 g.L$^{-1}$, 0.4 g.L$^{-1}$, 0.5 g.L$^{-1}$, 0.6 g.L$^{-1}$, 0.7 g.L$^{-1}$, 0.8 g.L$^{-1}$, 0.9 g.L$^{-1}$, 1 g.L$^{-1}$, 2 g.L$^{-1}$, 3 g.L$^{-1}$, 4 g.L$^{-1}$, 5 g.L$^{-1}$, 6 g.L$^{-1}$, 7 g.L$^{-1}$, 8 g.L$^{-1}$, 9 g.L$^{-1}$, 10 g.L$^{-1}$, 15 g.L$^{-1}$, 20 g.L$^{-1}$, 25 g.L$^{-1}$, 30 g.L$^{-1}$, 40 g.L$^{-1}$, 50 g.L$^{-1}$, 60 g.L$^{-1}$, 70 g.L$^{-1}$, 70 g.L$^{-1}$, 90 g.L$^{-1}$, 100 g.L$^{-1}$, 150 g.L$^{-1}$, 200 g.L$^{-1}$, 250 g.L$^{-1}$, 300 g.L$^{-1}$, 350 g.L$^{-1}$, 400 g.L$^{-1}$, 500 g.L$^{-1}$, 600 g.L$^{-1}$, 700 g.L$^{-1}$, 800 g.L$^{-1}$, 900 g.L$^{-1}$, 1000 g.L$^{-1}$. g.L$^{-1}$.

The transferrin-containing solution obtained by the process of the first or second aspect of the invention may be further purified and/or formulated for intravenous administration to a human and/or for provision as a cell culture ingredient and/or sterilised and/or placed into a final container.

It is preferred that the transferrin-containing solution obtained by the first or second aspect of the invention is suspended in a liquid, for example a solution of a buffer, suitable for final use of the transferrin. Such solutions include sodium chloride solutions, for example 145 mM NaCl.

The process of the first or second aspects of the invention may be preceded by one or more of the following steps: fermentation, primary separation, centrate conditioning, affinity separation, metal chelating separation, hydrophobic separation, liquid/liquid extraction, precipitation, ultrafiltration, size exclusion separation and/or mixed mode separation.

The transferrin may or may not be holoized prior to and/or following the cationic chromatographic step and/or the anionic chromatographic step of the first, second or third aspects of the invention. The transferrin may or may not be apoized prior to and/or following the cationic chromatographic step and/or the anionic chromatographic step of the first or second aspects of the invention. That is, the transferrin which is subjected to the purification process may be apo-transferrin, partially iron-saturated transferrin or holotransferrin.

Holoization and apoization techniques are known in the art. Transferrin may be holoized by the addition of sodium bicarbonate and iron (for example in the form of ammonium iron citrate) to about 2 moles of Fe$^{3+}$ per mole of transferrin and incubating at ambient temperature (*e.g.* about 20 to 25 °C) for at least one hour. Transferrin may be apoized at low pH in the presence of a chelating agent. For example transferrin may be apoized by the addition of 1 part transferrin solution to 9 parts of apoization solution and incubating the resultant mixture, with stirring, overnight at about 4 °C. Apoization solution may comprise 100 mM sodium acetate, 100 mM sodium citrate, 10 mM EDTA at pH 4.5.

The relatively impure transferrin may be obtained from a blood source or from a recombinant source such as prokaryotic (*e.g.* bacterial) or eukaryotic (*e.g.* fungal, animal, insect or plant). A preferred source of the relatively impure solution of transferrin is a microbial cell, such as a fungal cell (preferably a yeast cell such as *Saccharomyces (e.g.* S. *cerevisiae), Pichia (e.g. P. pastoris) or Kluyveromyces (e.g. K. lactis))* or a bacterial cell *(e.g.* a *Bacillus* or *Escherichia coli),* a mammalian cell (*e.g.* Chinese Hamster Ovary (CHO) or Baby Hamster Kidney (BHK) cell) or from blood or a blood fraction such as serum. The blood or blood fraction may be human or animal derived (such as bovine derived).

The 'transferrin' of the invention may be any protein with iron binding capacity which belongs to the transferrin family as described, *e.g.*, by Lambert et al., Comparative Biochemistry and Physiology, Part B, 142 (2005), 129-141, and by Testa, Proteins of iron metabolism, CRC Press, 2002; Harris & Aisen, Iron carriers and iron proteins, Vol. 5, Physical Bioinorganic Chemistry, VCH, 1991.

Examples of transferrin family proteins are serum transferrin, ovotransferrin, melanotransferrin and lactoferrin and their derivatives and variants, such as mutant transferrins (Mason et al., (1993) Biochemistry, 32, 5472; Mason et al., (1998), Biochem. J., 330, 35), truncated transferrins, transferrin lobes (Mason et al., (1996) Protein Expr. Purif., 8, 119; Mason et al., (1991) Protein Expr. Purif., 2, 214), mutant lactoferrins, truncated lactoferrins, lactoferrin lobes, mutant ovotransferrin, truncated ovotransferrin, melanotransferrin lobes, truncated melanotransferrin or fusions of any of the above to other peptides, polypeptides or proteins (Shin et al., (1995) Proc. Natl. Acad. Sci. USA, 92, 2820; Ali et al., (1999) J. Biol. Chem., 274, 24066; Mason et al., (2002) Biochemistry, 41, 9448). Serum transferrins are preferred, particularly a human serum transferrin (HST) having the amino acid sequence of SEQ ID NO: 1 with 679 amino acids, also called the C1 variant (Accession number NP_001054). Lactoferrins are preferred, particularly a human lactoferrin having the amino acid sequence of SEQ ID NO: 2 with 691 amino acids. Melanotransferrins are preferred, particularly human melanotransferrin having the amino acid sequence residue 20-738 of SEQ ID NO: 3.

The term 'transferrin' also includes both natural and engineered variants of transferrins for example fusions and conjugations of transferrins and conjugation-competent transferrins such as 'thio-transferrins'. Examples are described in WO2008/152140 and PCT/EP2008/060482. Conjugations of transferrin, for example 'thio-transferrins' may be prepared according to PCT/EP2008/060482 and "Protein Formulation and Delivery", E. J. McNally (Ed.), published by Marcel Dekker Inc. New York 2000 and "Rational Design of Stable Protein Formulations - Theory and Practice"; J. F. Carpenter and M. C. Manning (Ed.) Pharmaceutical Biotechnology Vol 13. Kluwer Academic/Plenum Publishers, New York 2002, Yazdi and Murphy, (1994) Cancer Research 54, 6387-6394, Widera et al., (2003) Pharmaceutical Research 20, 1231-1238; Lee et al., (2005) Arch. Pharm. Res. 28, 722-729. The transferrin of or purified by the claimed invention may or may not be a natural or engineered variant of transferrin as described herein. For example, the transferrin may or may not have one or more thiol groups available for conjugation to another molecule.

## [SEQ ID No. 1]

```
VPDKTVRWCA VSEHEATKCQ SFRDHMKSVI PSDGPSVACV KKASYLDCIR AIAANEADAV       60
TLDAGLVYDA YLAPNNLKPV VAEFYGSKED PQTFYYAVAV VKKDSGFQMN QLRGKKSCHT      120
GLGRSAGWNI PIGLLYCDLP EPRKPLEKAV ANFFSGSCAP CADGTDFPQL CQLCPGCGCS      180
TLNQYFGYSG AFKCLKDGAG DVAFVKHSTI FENLANKADR DQYELLCLDN TRKPVDEYKD      240
CHLAQVPSHT VVARSMGGKE DLIWELLNQA QEHFGKDKSK EFQLFSSPHG KDLLFKDSAH      300
GFLKVPPRMD AKMYLGYEYV TAIRNLREGT CPEAPTDECK PVKWCALSHH ERLKCDEWSV      360
NSVGKIECVS AETTEDCIAK IMNGEADAMS LDGGFVYIAG KCGLVPVLAE NYNKSDNCED      420
TPEAGYFAVA VVKKSASDLT WDNLKGKKSC HTAVGRTAGW NIPMGLLYNK INHCRFDEFF      480
SEGCAPGSKK DSSLCKLCMG SGLNLCEPNN KEGYYGYTGA FRCLVEKGDV AFVKHQTVPQ      540
NTGGKNPDPW AKNLNEKDYE LLCLDGTRKP VEEYANCHLA RAPNHAVVTR KDKEACVHKI      600
LRQQQHLFGS NVTDCSGNFC LFRSETKDLL FRDDTVCLAK LHDRNTYEKY LGEEYVKAVG      660
NLRKCSTSSL LEACTFRRP                                                   679
```

## SEQ ID No. 2]

```
GRRRSVQWCA VSQPEATKCF QWQRNMRKVR GPPVSCIKRD SPIQCIQAIA ENRADAVTLD       60
GGFIYEAGLA PYKLRPVAAE VYGTERQPRT HYYAVAVVKK GGSFQLNELQ GLKSCHTGLR      120
RTAGWNVPIG TLRPFLNWAG PPEPIEAAVA RFFSASCVPG ADKGQFPNLC RLCAGTGENK      180

CAFSSQEPYF SYSGAFKCLR DGAGDVAFIR ESTVFEDLSD EAERDEYELL CPDNTRKPVD      240
KFKDCHLARV PSHAVVARSV NGKEDAIWNL LRQAQEKFGK DKSPKFQLFG SPSGQKDLLF      300
KDSAIGFSRV PPRIDSGLYL GSGYFTAIQN LRKSEEEVAA RRARVVWCAV GEQELRKCNQ      360
WSGLSEGSVT CSSASTTEDC IALVLKGEAD AMSLDGGYVY TAGKCGLVPV LAENYKSQQS      420
SDPDPNCVDR PVEGYLAVAV VRRSDTSLTW NSVKGKKSCH TAVDRTAGWN IPMGLLFNQA      480
GSCKFDEYFS QSCAPGSDPR SNLCALCIGD EQGENKCVPN SNERYYGYTG AFRCLAENAG      540
DVAFVKDVTV LQNTDGNNNE AWAKDLKLAD FALLCLDGKR KPVTEARSCH LAMAPNHAVV      600
SRMDKVERLK QVLLHQQAKF GRNGADCPDK FCLFQSETKN LLFNDNTECL ARLHGKTTYE      660
KYLGPQYVAG ITNLKKCSTS PLLEACEFLR K                                     691
```

[SEQ ID No. 3]

```
MRGPSGALWL LLALRTVLGG MEVRWCATSD PEQHKCGNMS EAFREAGIQP SLLCVRGTSA     60
DHCVQLIAAQ EADAITLDGG AIYEAGKEHG LKPVVGEVYD QEVGTSYYAV AVVRRSSHVT    120
IDTLKGVKSC HTGINRTVGW NVPVGYLVES GRLSVMGCDV LKAVSDYFGG SCVPGAGETS    180
YSESLCRLCR GDSSGEGVCD KSPLERYYDY SGAFRCLAEG AGDVAFVKHS TVLENTDGKT    240
LPSWGQALLS QDFELLCRDG SRADVTEWRQ CHLARVPAHA VVVRADTDGG LIFRLLNEGQ    300
RLFSHEGSSF QMFSSEAYGQ KDLLFKDSTS ELVPIATQTY EAWLGHEYLH AMKGLLCDPN    360
RLPPYLRWCV LSTPEIQKCG DMAVAFRRQR LKPEIQCVSA KSPQHCMERI QAEQVDAVTL    420
SGEDIYTAGK TYGLVPAAGE HYAPEDSSNS YYVVAVVRRD SSHAFTLDEL RGKRSCHAGF    480
GSPAGWDVPV GALIQRGFIR PKDCDVLTAV SEFFNASCVP VNNPKNYPSS LCALCVGDEQ    540
GRNKCVGNSQ ERYYGYRGAF RCLVENAGDV AFVRHTTVFD NTNGHNSEPW AAELRSEDYE    600
LLCPNGARAE VSQFAACNLA QIPPHAVMVR PDTNIFTVYG LLDKAQDLFG DDHNKNGFKM    660
FDSSNYHGQD LLFKDATVRA VPVGEKTTYR GWLGLDYVAA LEGMSSQQCS GAAAPAPGAP    720
LLPLLLPALA ARLLPPAL                                                 738
```

A particularly preferred transferrin consists of or comprises the amino acid sequence of SEQ ID No. 4:

[SEQ ID No. 4]

```
VPDKTVRWCA VSEHEATKCQ SFRDHMKSVI PSDGPSVACV KKASYLDCIR AIAANEADAV     60
TLDAGLVYDA YLAPNNLKPV VAEFYGSKED PQTFYYAVAV VKKDSGFQMN QLRGKKSCHT    120
GLGRSAGWNI PIGLLYCDLP EPRKPLEKAV ANFFSGSCAP CADGTDFPQL CQLCPGCGCS    180
TLNQYFGYSG AFKCLKDGAG DVAFVKHSTI FENLANKADR DQYELLCLDN TRKPVDEYKD    240
CHLAQVPSHT VVARSMGGKE DLIWELLNQA QEHFGKDKSK EFQLFSSPHG KDLLFKDSAH    300
GFLKVPPRMD AKMYLGYEYV TAIRNLREGT CPEAPTDECK PVKWCALSHH ERLKCDEWSV    360
NSVGKIECVS AETTEDCIAK IMNGEADAMS LDGGFVYIAG KCGLVPVLAE NYNKADNCED    420
TPEAGYFAVA VVKKSASDLT WDNLKGKKSC HTAVGRTAGW NIPMGLLYNK INHCRFDEFF    480
SEGCAPGSKK DSSLCKLCMG SGLNLCEPNN KEGYYGYTGA FRCLVEKGDV AFVKHQTVPQ    540
NTGGKNPDPW AKNLNEKDYE LLCLDGTRKP VEEYANCHLA RAPNHAVVTR KDKEACVHKI    600
LRQQQHLFGS NVADCSGNFC LFRSETKDLL FRDDTVCLAK LHDRNTYEKY LGEEYVKAVG    660
NLRKCSTSSL LEACTFRRP                                                679
```

Preferably the transferrin which undergoes purification does not contain a leader sequence. For example, a transferrin may be produced with a leader sequence and the leader sequence may or may not be removed prior to the purification. Alternatively, the transferrin may be produced without the leader sequence. Examples of transferrin sequences without a leader sequence are given in SEQ ID No. 1 and SEQ ID No. 4.

The transferrin may be modified relative to a naturally occurring transferrin or to a known engineered variant of transferrin. For example, the transferrin may consist of or comprise an amino acid sequence which has at least 25% identity to SEQ ID NO: 1, SEQ ID No. 2 or SEQ ID No. 3 or SEQ ID No. 4, particularly at least 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9%. More particularly, it may have 100% identity to SEQ ID No. 1, SEQ ID No. 2, SEQ ID No. 3 or most particularly SEQ ID No. 4.

The amino acid sequence of the transferrin family protein may have a length of at least 167 amino acids, particularly at least 300 amino acids, 630 amino acids, 691 amino acids, 694 amino acids or 695 amino acids. The length is typically at most 1274 amino acids, particularly at most 819 amino acids, at most 722 amino acids, at most 717 amino acids or at most 706 amino acids. The length may particularly be 679 amino acids. It may be a vertebrate transferrin with 691-717 amino acids or a mammalian transferrin with a length of 695-706 amino acids.

The transferrin family protein, particularly one having a length of 694-706 amino acids, generally contains two domains (called the N- and C-lobes), each having around 331-341 residues and each binding one atom of Fe (III) and one carbonate anion, connected by an inter-lobe linker of about 7 residues. Typically, each iron is coordinated to four conserved amino acid residues: one Asp, two Tyr and one His, and the carbonate anion is bound to an Arg and a Thr.

Preferably, the transferrin family protein purified in the invention has at least 40% or at least 60% identity to full-length HST (residues 1-679 of SEQ ID NO: 1) or to the C-lobe of HST (residues 339-679 of SEQ ID NO. 1). The primary receptor-recognition site of HST for the TfR is in the C-lobe, and proteolytically isolated HST C-lobe is able to deliver ferric iron to cells.

The human serum transferrin may be the variant designated $TfC_1$, $TfC_2$ or $TfC_3$.

A number of proteins are known to exist within the transferrin family and a non-exclusive list is shown below. The list indicates the full length of the sequences, including the mature protein and the leader sequence. Each of these transferrins, and variants thereof, may or may not be purified by the process of the invention.

**Table 1: Examples of transferrins**

| Protein | Species | Common Name | Accession Number | Identity to SEQ ID NO: 1 | Length |
|---|---|---|---|---|---|
| Transferrin | *Homo sapiens* | Human | NP_001054 | 100 % | 698 aa |
| Transferrin | *Canis lupus familiaris* | Dog | XP_864515 | 73.2 | 706 aa |
| Transferrin | *Mus musculus* | House mouse | NP_598738 | 73.7 | 697 aa |
| Transferrin | *Rattus norvegicus* | Norway rat | NP_001013128 | 73.6 | 698 aa |
| Transferrin | *Sus scrofa* | Pig | CAA30943 | 71.6 | 696 aa |
| Transferrin | *Oryctolagus cuniculus* | Rabbit | AAB94136 | 79.0 | 695 aa |
| Transferrin | *Equus caballus* | Horse | NP_001075415 | 73.7 | 706 aa |
| Transferrin | *Bos taurus* | Cattle | NP_803450 | 70.1 | 704 aa |
| Transferrin | *Gallus gallus* | Chicken | NP_990635 | 53.1 | 705 aa |
| Transferrin | *Marmota monax* | Woodchuck | AAP37129 | | 694 aa |
| Transferrin | *Xenopus laevis* | African clawed frog | NP_001079812 | | 717 aa |
| Transferrin | *Xenopus tropicalis* | African clawed frog | NP_001027487 | 49.6 | 703 aa |
| Transferrin | *Chamaeleo calyptratus* | Veiled Chameleons | CAK18229 | | 710 aa |
| Transferrin | *Lacerta agilis* | Sand Lizard | CAK18228 | | 714 aa |
| Transferrin | *Eublepharis macularius* | Leopard gecko | CAK18227 | | 703 aa |
| Transferrin | *Pogona vitticeps* | Central bearded dragon | CAK18226 | | 702 aa |
| Transferrin | *Anolis sagrei* | Brown anole | CAK18225 | | 710 aa |
| Transferrin | *Lamprophis fuliginosus* | African House Snake | CAK18223 | | 711 aa |
| Transferrin | *Natrix natrix* | Grass Snake | CAK18221 | 50.0 | 710 aa |
| Transferrin | *Oncorhynchus mykiss* | Rainbow trout | BAA84103 | | 691 aa |
| Transferrin | *Oryzias latipes* | Japanese medaka | BAA10901 | | 690 aa |
| Transferrin | *Oreochromis niloticus* | Nile tilapia | ABB70391 | | 694 aa |

(continued)

| Protein | Species | Common Name | Accession Number | Identity to SEQ ID NO: 1 | Length |
|---|---|---|---|---|---|
| Transferrin | *Oncorhynchus tshawytscha* | Chinook salmon | AAF03084 | | 672 aa |
| Transferrin | *Gadus morhua* | Atlantic cod | AAB08440 | | 642 aa |
| Transferrin | *Salmo trutta* | Brown trout | BAA84102 | | 691 aa |
| Transferrin | *Salvelinus namaycush* | Lake trout | BAA84101 | | 691 aa |
| Transferrin | *Salvelinus fontinalis* | Brook trout | BAA84100 | | 691 aa |
| Transferrin | *Salvelinus pluvius* | Japanese fish | BAA84099 | | 691 aa |
| Transferrin | *Oncorhynchus masou* | Cherry salmon | BAA84098 | | 691 aa |
| Transferrin | *Oncorhynchus rhodurus* | Amago | BAA84097 | | 691 aa |
| Transferrin | *Oncorhynchus nerka* | Sockeye salmon | BAA84096 | 50.2 | 691 aa |
| Transferrin | *Paralichthys olivaceus* | Bastard halibut | BAA28944 | | 685 aa |
| Transferrin | *Oncorhynchus kisutch* | Coho salmon | BAA13759 | | 687 aa |
| Transferrin | *Oreochromis aureus* | Tilapia (cichlid) | CAC59954 | | 167 aa |
| Transferrin | *Danio rerio* | Zebrafish | DAA01798 | | 675 aa |
| Transferrin | *Pagrus major* | Red seabream | AAP94279 | | 691 aa |
| Melanotransferrin | *Homo sapiens* | Human | NP_005920 | 45.7 | 738 aa |
| Meanotransferrin | *Canis lupus familiaris* | Dog | XP_545158 | 42.3 | 1193 aa |
| Melanotransferrin | *Mus musculus* | House mouse | NP_038928 | 44.4 | 738 aa |
| Melanotransferrin | *Rattus norvegicus* | Norway rat | XP_237839 | 44.7 | 738 aa |
| Melanotransferrin | *Gallus gallus* | Chicken | NP_990538 | 43.6 | 738 aa |
| Lactotransferrin | *H. sapiens* | Human | NP_002334 | 61.8 | 710 aa |
| Lactotransferrin | *Pan troglodytes* | Chimpanzee | XP_516417 | 62.0 | 711 aa |
| Lactotransferrin | *Canis lupus familiaris* | Dog | XP_864480 | 61.9 | 724 aa |
| Lactotransferrin | *Mus musculus* | House mouse | NP_032548 | 57.6 | 707 aa |
| Lactotransferrin | *Rattus norvegicus* | Norway rat | XP_236657 | 53.8 | 729 aa |
| Lactoferrin | *Sus scrofa* | Pig | AAA31059 | 61.1 | 703 aa |
| Lactoferrin | *Camelus dromedarius* | Arabian carnel | CAB53387 | 62.0 | 708 aa |
| Lactoferrin | *Equus caballus* | Horse | CAA09407 | 63.7 | 695 aa |

(continued)

| Protein | Species | Common Name | Accession Number | Identity to SEQ ID NO: 1 | Length |
|---|---|---|---|---|---|
| Lactoferrin | *Bos taurus* | Cattle | AAA30610 | 62.0 | 708 aa |
| Lactoferrin | *Bubalus bubalis* | Water buffalo | CAA06441 | 62.1 | 708 aa |
| Lactoferrin | *Capra hircus* | Goat | ABD49106 | 62.2 | 708 aa |
| Lactoferrin | *Bos grunniens* | Domestic yak | ABD49105 | 62.0 | 708 aa |
| Lactoferrin | *Ovis aries* | Sheep | AAV92908 | 62.4 | 708 aa |
| Ovotransferrin | *Anas platyrhynchos* | Mallard duck | P56410 | 54.4 | 686 aa |
| Ovotransferrin | *Gallus gallus* | Chicken | CAA26040 | 53.1 | 705 aa |

The transferrin may or may not be fused and/or conjugated to a protein or other molecule such as a bioactive compound. The protein or other molecule, such as a bioactive compound, may or may not have a therapeutic and/or diagnostic use. In this context, 'fusion' includes a protein in which the transferrin and the other protein are encoded by the same DNA sequence whereas 'conjugation' includes a protein in which the transferrin has a protein or other molecule bound to it through any other means, for example through chemical conjugation. US 20030221201 and 20040023334 describe fusion proteins comprising a transferrin protein fused to a therapeutic protein. The transferrin may or may not be both a fused and conjugated transferrin.

The transferrin may or may not be a therapeutic or diagnostic transferrin. A 'therapeutic transferrin' is a transferrin which is suitable for use in preventative therapy or for curative therapy, for example treatment of anaemia. A 'diagnostic transferrin' is a transferrin which is suitable for use in diagnosis, for example the transferrin may be fused or conjugated to an antibody or to a label such as label or contrast agent suitable for use in imaging. The diagnostic transferrin may be used *in vivo* or *in vitro.*

The therapeutic transferrin may comprise a chemotherapy drug for use in cancer chemotherapy. It may be cytostatic or cytotoxic; it may be a tumor-inhibiting agent.

The bioactive compound may be a polypeptide (protein), particularly a recombinant protein pharmaceutical. It may be a chemotherapy or radiotherapy drug used to treat cancers and/or other related diseases.

The bioactive compound may be conjugated to a free thiol of a transferrin. The bioactive compound includes but is not limited to, peptides, polypeptides or proteins (either natural, recombinant, or synthetic). The free thiol may be on a cysteine residue.

A third aspect of the invention comprises a process for producing a transferrin comprising expressing a transferrin, for example in cells, particularly in fungal cells such as yeast cells, and purifying the resultant transferrin in accordance with the first, or second aspect of the invention.

A fourth aspect of the invention comprises the step of lyophilising the transferrin purified by the first, second, or third aspect of the invention.

A fifth aspect provides a purified transferrin obtained or obtainable by a process according to the first, second, third, or fourth aspect of the invention.

A sixth aspect relates to the use of a transferrin according to the first, second, third, fourth, or fifth aspect in therapy (preventative and/or curative) or diagnosis. For example, the transferrin may be used in leukaemia treatment to prevent the harmful effects of non-transferrin-bound iron in a patient. The transferrin, for example a fused or conjugated transferrin, may be used in targeting, for example in diagnosis, imaging, localization and/or treatment of tumours.

A seventh aspect relates to the use of a transferrin according to the first, second, third, fourth, or fifth aspect in cell culture such as mammalian cell culture, particularly human cell culture. Cell culture may be at laboratory or industrial scale. The disclosure also relates to a cell culture medium comprising the transferrin according to the first, second, third, fourth, fifth or sixth aspect. The disclosure also includes manufacture of such a cell culture medium.

An eighth aspect provides a pharmaceutical composition comprising a transferrin purified according to the first, second, third, or fourth aspect and a pharmaceutically acceptable carrier and/or diluent. Therefore, the purified transferrin may be formulated with a carrier or diluent. The thus formulated transferrin may optionally be presented in a unit dosage form. The carrier(s) or diluent(s) must be "acceptable" in the sense of being compatible with the desired protein. Typically, the carriers or diluents will be water or saline which will be sterile and pyrogen free. The disclosure also includes provision of a transferrin according to the aspects for use as a pharmaceutical ingredient. The transferrin may or may not be fused or conjugated to another molecule which may or may not be a bioactive molecule.

With regards the 'starting material' (i.e. the relatively impure transferrin solution from which the transferrin is purified by the process of the invention), the transferrin or fusions of transferrin and another protein or proteins can be prepared by methods know in the art (Sanker, (2004), Genetic Eng. News, 24, 22-28, Schmidt, (2004), Appl. Microbiol. Biotechnol., 65, 363-372) including but not limited to expression in mammalian cell culture (Mason et al., (2004), Protein Expr. Purif., 36, 318-326; Mason et al., (2002), Biochemistry, 41, 9448-9454) from cells lines such as CHO (and its variants), NSO, BHK, HEK293, Vero or PERC6 cells by transformation or transient expression; insect cell culture (Lim et al., (2004) Biotechnol. Prog., 20, 1192-1197); plant cell culture from such plants as Lemna; transgenic animals (Dyck et al., (2003) Trends in Biotechnology, 21, 394-399); transgenic plants (Ma et al., (2003) Nature Reviews Genetics, 4, 794-805); Gram positive and Gram negative bacteria such as *Bacillus* and *Escherichia coli* (Steinlein, and Ikeda, (1993), Enzyme Microb. Technol., 15, 193-199); filamentous fungi including but not restricted to *Aspergillus spp* (EP 238023, US 5,364,770, US 5,578,463, EP184438, EP284603, WO 2000/056900, WO9614413), *Trichoderma* spp and *Fusarium* spp (Navalainen et al., (2005), Trends in Biotechnology, 23, 468-473).

Polypeptides which are variants of full-length HST may be expressed recombinantly from baby hamster kidney (BHK) cells (Mason et al., (2004), Protein Expr. Purif., 36, 318-326, Mason et al., (2002), Biochemistry, 41, 9448-9454), *D. melanogaster* S2 cells (Lim et al., (2004), Biotechnol Prog., 20, 1192-1197) and as a non-N-linked glycosylated mutant from BHK cells (Mason et al., (2001), Protein Expr. Purif., 23, 142-150, Mason et al., (1993), Biochemistry, 32, 5472-5479) and *S. cerevisiae* (Sargent et al., (2006), Biometals 19, 513-519). The polypeptides which are variants of C-lobe of HST (NTf/2C) may be expressed from BHK cells (Mason et al., (1997), Biochem. J., 326 (Pt 1), 77-85). In one embodiment the host cell is a yeast cell, such as a member of the *Saccharomyces, Kluyveromyces,* or *Pichia* genus, such as *Sacharomyces cerevisiae, Kluyveromyces lactis, Pichia pastoris* (Mason et al., (1996), Protein Expr. Purif., 8, 119-125, Steinlein et al., 1995 Protein Expr. Purif., 6, 619-624), *Pichia methanolica* (Mayson et al., (2003) Biotechnol. Bioeng., 81, 291-298) and *Pichia membranaefaciens,* or *Zygosaccharomyces rouxii* (formerly classified as *Zygosaccharomyces bisporus), Zygosaccharomyces bailii, Zygosaccharomyces fermentati, Hansenula polymorpha* (also known as *Pichia angusta)* or *Kluyveromyces drosophilarum* are preferred.

The host cell may be any type of cell. The host cell may or may not be an animal (such as mammalian, avian, insect, etc.), plant, fungal or bacterial cell. Bacterial and fungal, such as yeast, host cells may or may not be preferred.

Typical prokaryotic vector plasmids are: pUC18, pUC19, pBR322 and pBR329 available from BioRad Laboratories (Richmond, CA, USA); p*Trc*99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA).

A typical mammalian cell vector plasmid is pSVL available from Pharmacia (Piscataway, NJ, USA). This vector uses the SV40 late promoter to drive expression of cloned genes, the highest level of expression being found in T antigen-producing cells, such as COS-1 cells. An example of an inducible mammalian expression vector is pMSG, also available from Pharmacia (Piscataway, NJ, USA). This vector uses the glucocorticoid-inducible promoter of the mouse mammary tumour virus long terminal repeat to drive expression of the cloned gene.

Methods well known to those skilled in the art can be used to construct expression vectors containing the coding sequence of a desired protein and, for example appropriate transcriptional or translational controls. One such method involves ligation via cohesive ends. Compatible cohesive ends can be generated on the DNA fragment and vector by the action of suitable restriction enzymes. These ends will rapidly anneal through complementary base pairing and remaining nicks can be closed by the action of DNA ligase.

A further method uses synthetic double stranded oligonucleotide linkers and adaptors. DNA fragments with blunt ends are generated by bacteriophage T4 DNA polymerase or *E. coli* DNA polymerase I which remove protruding 3' termini and fill in recessed 3' ends. Synthetic linkers and pieces of blunt-ended double-stranded DNA which contain recognition sequences for defined restriction enzymes, can be ligated to blunt-ended DNA fragments by T4 DNA ligase. They are subsequently digested with appropriate restriction enzymes to create cohesive ends and ligated to an expression vector with compatible termini. Adaptors are also chemically synthesised DNA fragments which contain one blunt end used for ligation but which also possess one preformed cohesive end. Alternatively a DNA fragment or DNA fragments can be ligated together by the action of DNA ligase in the presence or absence of one or more synthetic double stranded oligonucleotides optionally containing cohesive ends.

Synthetic linkers containing a variety of restriction endonuclease sites are commercially available from a number of sources including Sigma-Genosys Ltd, London Road, Pampisford, Cambridge, United Kingdom.

The transferrin or fusions of transferrin and another protein or proteins will be expressed from a nucleotide sequence, which may or may not contain one or more introns. Additionally the nucleotide sequence may or may not be codon optimised for the host by methods known to the art.

The transferrin or fusions of transferrin and another protein or proteins can be expressed as variants with reduced N-linked glycosylation. Accordingly, in case of human serum transferrin (HST), N413 can be changed to any amino acid, preferably, Q, D, E or A; S415 can be changed to any amino acid except S or T, preferably, A; T613 can be changed to any amino acid except S or T, preferably, A; N611 can be changed to any amino acid; or combinations of the above. Where the transferrin is not HST, reduction in N-glycosylation can be achieved by similar modification to the protein primary sequence. For clarity, the transferrin or fusions of transferrin and another protein or proteins can be both a transferrin variant of the invention and have reduced N-linked glycosylation.

It may be particularly advantageous to use a host, for example a yeast, deficient in one or more protein mannosyl transferases involved in O-glycosylation of proteins, for instance by disruption of the gene coding sequence. Recombinantly expressed proteins can be subject to undesirable post-translational modifications by the producing host cell. The mannosylated transferrin would be able to bind to the lectin Concanavalin A. The amount of mannosylated transferrin produced by the yeast can be reduced by using a yeast strain deficient in one or more of the *PMT* genes (WO 94/04687). The most convenient way of achieving this is to create a yeast which has a defect in its genome such that a reduced level of one of the Pmt proteins is produced. For example, there may or may not be a deletion, insertion or transposition in the coding sequence or the regulatory regions (or in another gene regulating the expression of one of the *PMT* genes) such that little or no Pmt protein is produced. Alternatively, the yeast could be transformed to produce an anti-Pmt agent, such as an anti-Pmt antibody. Alternatively, the yeast could be cultured in the presence of a compound that inhibits the activity of one of the PMT genes (Duffy et al, "Inhibition of protein mannosyltransferase 1 (PMT1) activity in the pathogenic yeast Candida albicans", International Conference on Molecular Mechanisms of Fungal Cell Wall Biogenesis, 26-31 August 2001, Monte Verita, Switzerland, Poster Abstract P38; the poster abstract may be viewed at http://www.micro.biol.ethz.ch/cellwall/). If a yeast other than *S. cerevisiae* is used, disruption of one or more of the genes equivalent to the *PMT* genes of *S. cerevisiae* is also beneficial, *e.g.* in *Pichia pastoris* or *Kluyveromyces lactis.* The sequence of *PMT1* (or any other *PMT* gene) isolated from *S. cerevisiae* may be used for the identification or disruption of genes encoding similar enzymatic activities in other fungal species. The cloning of the *PMT1* homologue of *Kluyveromyces lactis* is described in WO 94/04687.

The yeast may or may not also have a deletion of the *HSP150* and/or *YAP3* genes as taught respectively in WO 95/33833 and WO 95/23857.

The transferrin may be produced by recombinant expression and secretion. Where the expression system (*i.e.* the host cell) is yeast, such as *Saccharomyces cerevisiae,* suitable promoters for S. *cerevisiae* include those associated with the *PGK1* gene, *GAL1* or *GAL10* genes, *TEF1, TEF2, PYK1, PMA1, CYC1, PHO5, TRP1, ADH1, ADH2,* the genes for glyceraldehyde-3-phosphate dehydrogenase, hexokinase, pyruvate decarboxylase, phosphofructokinase, triose phosphate isomerase, phosphoglucose isomerase, glucokinase, $\alpha$-mating factor pheromone, $\alpha$-mating factor pheromone, the *PRB1* promoter, the *PRA1* promoter, the *GPD1* promoter, and hybrid promoters involving hybrids of parts of 5' regulatory regions with parts of 5' regulatory regions of other promoters or with upstream activation sites (e.g. the promoter of EP-A-258 067).

Suitable transcription termination signals are well known in the art. Where the host cell is eukaryotic, the transcription termination signal is preferably derived from the 3' flanking sequence of a eukaryotic gene, which contains proper signals for transcription termination and polyadenylation. Suitable 3' flanking sequences may, for example, be those of the gene naturally linked to the expression control sequence used, *i.e.* may correspond to the promoter. Alternatively, they may be different. In that case, and where the host is a yeast, preferably S. *cerevisiae,* then the termination signal of the *S. cerevisiae ADH1, ADH2, CYC1,* or *PGK1* genes are preferred.

It may be beneficial for the promoter and open reading frame of the gene encoding the recombinant protein comprising the sequence of a transferrin mutant to be flanked by transcription termination sequences so that the transcription termination sequences are located both upstream and downstream of the promoter and open reading frame, in order to prevent transcriptional read-through into any neighbouring genes, such as $2\mu$m genes, and vice versa.

In one embodiment, the favoured regulatory sequences in yeast, such as Saccha*romyces cerevisiae,* include: a yeast promoter (*e.g.* the *Saccharomyces cerevisiae PRB1* promoter), as taught in EP 431 880; and a transcription terminator, preferably the terminator from *Saccharomyces ADH1,* as taught in EP 60 057.

It may be beneficial for the non-coding region to incorporate more than one DNA sequence encoding a translational stop codon, such as UAA, UAG or UGA, in order to minimise translational read-through and thus avoid the production of elongated, non-natural fusion proteins. The translation stop codon UAA is preferred.

It is preferred that the recombinant protein comprising the sequence of a transferrin mutant is secreted. In that case, a sequence encoding a secretion leader sequence may be included in the open reading frame. Thus, a polynucleotide according to the present invention may comprise a sequence that encodes a recombinant protein comprising the sequence of a transferrin mutant operably linked to a polynucleotide sequence that encodes a secretion leader sequence. Leader sequences are usually, although not necessarily, located at the N-terminus of the primary translation product of an ORF and are generally, although not necessarily, cleaved off the protein during the secretion process, to yield the "mature" protein. Thus, in one embodiment, the term "operably linked" in the context of leader sequences includes the meaning that the sequence that encodes a recombinant protein comprising the sequence of a transferrin mutant is linked, at its 5' end, and in-frame, to the 3' end of a polynucleotide sequence that encodes a secretion leader sequence. Alternatively, the polynucleotide sequence that encodes a secretion leader sequence may be located, in-frame, within the coding sequence of the recombinant protein comprising the sequence of a transferrin mutant, or at the 3' end of the coding sequence of the recombinant protein comprising the sequence of a transferrin mutant.

Numerous natural or artificial polypeptide leader sequences (also called secretion pre regions and pre/pro regions) have been used or developed for secreting proteins from host cells. Leader sequences direct a nascent protein towards the machinery of the cell that exports proteins from the cell into the surrounding medium or, in some cases, into the periplasmic space.

For production of proteins in eukaryotic species such as the yeasts *Saccharomyces cerevisiae, Zygosaccharomyces* species, *Kluyveromyces lactis* and *Pichia pastoris,* a secretion leader sequence may be used. This may comprise a signal (pre) sequence or a prepro leader sequence. Signal sequences are known to be heterogeneous in their amino acid sequence (Nothwehr and Gordon 1990, Bioessays 12, 479-484, or Gierasch 1989, Biochemistry 28, p923-930). In essence, signal sequences are generally N-terminally located, have a basic n-region, a hydrophobic h-region and a polar c-region. As long as this structure is retained the signal sequence will work, irrespective of the amino acid composition. How well they work, *i.e.* how much mature protein is secreted, depends upon the amino acid sequence. Accordingly, the term "signal peptide" is understood to mean a presequence which is predominantly hydrophobic in nature and present as an N-terminal sequence of the precursor form of an extracellular protein expressed in yeast. The function of the signal peptide is to allow the expressed protein to be secreted to enter the endoplasmic reticulum. The signal peptide is normally cleaved off in the course of this process. The signal peptide may be heterologous or homologous to the yeast organism producing the protein. Known leader sequences include those from the S. *cerevisiae* acid phosphatase protein (Pho5p) (see EP 366 400), the invertase protein (Suc2p) (see Smith et al. (1985) Science, 229, 1219-1224) and heat-shock protein-150 (Hsp150p) (see WO 95/33833). Additionally, leader sequences from the S. *cerevisiae* mating factor alpha-1 protein (MF$\alpha$-1) and from the human lysozyme and human serum albumin (HSA) protein have been used, the latter having been used especially, although not exclusively, for secreting human albumin. WO 90/01063 discloses a fusion of the MF$\alpha$-1 and HSA leader sequences. In addition, the natural transferrin leader sequence may or may not be used to direct secretion of the recombinant protein comprising the sequence of a transferrin mutant.

The skilled person will appreciate that any suitable plasmid may be used, such as a centromeric plasmid. The examples provide suitable plasmids (centromeric YCplac33-based vectors) for use to transform yeast host cells of the present invention. Alternatively, any other suitable plasmid may be used, such as a yeast-compatible 2$\mu$m-based plasmid.

Plasmids obtained from one yeast type can be maintained in other yeast types (Irie et al, 1991, Gene, 108(1), 139-144; Irie et al, 1991, Mol. Gen. Genet., 225(2), 257-265). For example, pSR1 from *Zygosaccharomyces rouxii* can be maintained in *Saccharomyces cerevisiae.* In one embodiment the plasmid may or may not be a 2$\mu$m-family plasmid and the host cell will be compatible with the 2$\mu$m-family plasmid used (see below for a full description of the following plasmids). For example, where the plasmid is based on pSR1, pSB3 or pSB4 then a suitable yeast cell is *Zygosaccharomyces rouxii;* where the plasmid is based on pSB1 or pSB2 then a suitable yeast cell is *Zygosaccharomyces bailli;* where the plasmid is based on pSM1 then a suitable yeast cell is *Zygosaccharomyces fermentati;* where the plasmid is based on pKD1 then a suitable yeast cell is *Kluyveromyces drosophilarum;* where the plasmid is based on pPM1 then a suitable yeast cell is *Pichia membranaefaciens;* where the plasmid is based on the 2$\mu$m plasmid then a suitable yeast cell is *Saccharomyces cerevisiae* or *Saccharomyces carlsbergensis.* Thus, the plasmid may be based on the 2$\mu$m plasmid and the yeast cell may be *Saccharomyces cerevisiae.* A 2$\mu$m-family plasmid can be said to be "based on" a naturally occurring plasmid if it comprises one, two or preferably three of the genes *FLP, REP1* and *REP2* having sequences derived from that naturally occurring plasmid.

Useful yeast episomal plasmid vectors are pRS403-406 and pRS413-416 and are generally available from Stratagene Cloning Systems (La Jolla, CA 92037, USA), YEp24 (Botstein, D., et al. (1979) Gene 8, 17-24), and YEplac122, YEplac195 and YEplac181 (Gietz, R.D. and Sugino. A. (1988) Gene 74, 527-534). Other yeast plasmids are described in WO 90/01063 and EP 424 117, as well as the "disintegration vectors of EP-A-286 424 and WO2005061719. Plasmids pRS403, pRS404, pRS405 and pRS406 are Yeast Integrating plasmids (YIps) and incorporate the yeast selectable markers *HIS3, TRP1, LEU2* and *URA3,* as are YIplac204, YIplac211 and YIplac128 (Gietz, R.D. and Sugino. A. (1988) Gene 74, 527-534). Plasmids pRS413-416 are Yeast Centromere plasmids (YCps) as are YCplac22, YCplac33 and YCplac111 (Gietz, R.D. and Sugino. A. (1988) Gene 74, 527-534).

The processes of the present invention can be used to obtain highly purified transferrin from a relatively impure transferrin solution from a number of sources such as blood products and eukaryotic or prokaryotic cell culture. However, the processes are particularly applicable to purifying recombinant transferrin (rTf), particularly from a yeast such as S. cerevisiae. The transferrin produced in accordance with the invention may be any mammalian transferrin such as bovine transferrin, but it is preferably human transferrin or a derivative thereof.

The invention is described, by way of example only, with reference to the drawings and the following, non-limiting, examples:

**EXAMPLES**

**EXAMPLE 1: Cation exchange low pH / high salt wash**

**Recovery data obtained for washing Sepharose Fast Flow (SP-FF) with nine different wash buffers (pH / sodium chloride concentration combinations)**

(a) Low pH/High Salt Wash

Initial Optimization: Twelve runs were performed on an AKTA™ Explorer 100 Air chromatography system (GE Healthcare) on an 8.6 mL column. Washing was carried out using different combinations of pH and salt concentration. Specifically, pH 4.0, 4.5 and 5.1 and sodium chloride concentrations of 0.25, 1.0 and 2.0 M were used. Start material (i.e. solution of about 3.7 mg/ml transferrin) was thawed and conditioned to pH 5.1; 3.5 mS.cm$^{-1}$ by adding acetic acid. The column was equilibrated at pH 5.0-5.2 and 2.5-3.1 mS.cm$^{-1}$, conditioned material was loaded at 25 mg transferrin.mL matrix$^{-1}$ and washed first with buffer of the appropriate pH (i.e. the same pH the wash to be used in wash 2) and no sodium chloride (wash 1). The column was then washed with the low pH/high salt buffer (wash 2) and bound transferrin was eluted with elution buffer (50 mM sodium phosphate, 25 mM sodium chloride, pH 7.0). Load, flow through, wash and eluate fractions were collected, and their transferrin content assayed by GP.HPLC using an area calibration method. This data was used to calculate mass balance and recovery values. Load, wash 2 and eluate fractions were also subjected to yeast antigen (HCP) ELISA analysis, and yeast antigen (HCP) clearance values were calculated.

**Table 2: Affect of wash conditions on recovery of transferrin in eluate**

| Run number | Wash conditions | | Eluate recovery (%) |
|---|---|---|---|
| | NaCl (M) | pH | |
| 1 | 0.25 | 4.0 | 53.9 |
| 2 | 1.0 | 4.0 | 38.7 |
| 3 | 2.0 | 4.0 | 48.4 |
| 4 | 0.25 | 4.5 | 87.2 |
| 5 | 1.0 | 4.5 | 76.3 |
| 6 | 2.0 | 4.5 | 94.0 |
| 7 | 0.25 | 4.5 | 82.1 |
| 8 | 1.0 | 4.5 | 74.1 |
| 9 | 2.0 | 4.5 | 79.7 |
| 10 | 0.25 | 5.1 | 15.2 |
| 11 | 1.0 | 5.1 | 15.9 |
| 12 | 2.0 | 5.1 | 31.7 |

These data (Table 2) show that, of the conditions tested, pH 4.5 at any sodium chloride concentration gives the best recoveries and that at pH 4.5, 2.0 M sodium chloride is the optimum.

**Table 3: Mean recovery and yeast antigen (HCP) clearance data for washing SP-FF with three different pH/ sodium chloride concentration combinations**

| Wash buffer | | Mean eluate recovery (%) | Mean yeast antigen (HCP) clearance (fold) |
|---|---|---|---|
| NaCl (M) | pH | | |
| 0.25 | 4.5 | 84.7 | 24.6 |
| 1 | 4.5 | 75.2 | 27.8 |
| 2 | 4.5 | 86.9 | 25.5 |

Data is a mean of two runs for each sodium chloride concentration. Eluate recovery data is from Table 2. This shows that the wash buffer having 2 M sodium chloride at pH 4.5 gives the best eluate recovery/yeast antigen (HCP) clearance combination.

(b) Further Optimization

This example shows the affect of low pH / high salt wash on yeast antigen (HCP) clearance and transferrin concentration of the eluate.

An SP-FF run was performed on the AKTA Explorer 100 Air on an 8.6 mL column using the pH 4.5, 2 M sodium chloride wash 2 conditions detailed above but with the addition of a third wash step using the equilibration buffer. A further three runs were performed to compare the following three wash regimes:

A: Wash 1 (50 mM sodium acetate pH 5.1)
B: Wash 1 (50 mM sodium acetate pH 4.5); Wash 2 (27 mM sodium acetate, 2 M sodium chloride pH 4.5)
C: Wash 1 (50 mM sodium acetate pH 4.5); Wash 2 (27 mM sodium acetate, 2 M sodium chloride pH 4.5); Wash 3 (50 mM sodium acetate pH 5.1)

Samples from throughout the purifications were subjected to GP.HPLC and ELISA analysis as described above and the data used to calculate transferrin recovery and yeast antigen (HCP) clearance values.

**Table 4: Recovery and yeast antigen (HCP) clearance data comparing the three different SP-FF wash regimes**

| | SP-FF wash regime | Eluate recovery (%) | Yeast antigen (HCP) clearance (fold) |
|---|---|---|---|
| A | Wash 1: 50 mM sodium acetate pH 5.1 | 72.1 | 16.8 |
| B | Wash 1: 27 mM sodium acetate pH 4.5<br>Wash 2: 50 mM sodium acetate, 2 M sodium chloride pH 4.5 | 70.9 | 27.6 |
| C | Wash 1: 50 mM sodium acetate pH 4.5<br>Wash 2: 27 mM sodium acetate, 2 M sodium chloride pH 4.5<br>Wash 3: 50 mM sodium acetate pH 5.1 | 67.5 | 30.1 |

These data (Table 4) show that the two-wash regime (B) gives a significantly higher yeast antigen (HCP) clearance than the single-wash regime (A) and that the three-wash regime (C) improves on this further with only a relatively small reduction in eluate recovery.

**EXAMPLE 2: Affect of presence of borate in anion exchange chromatography load buffer on the recovery of** transferrin **from the Q-FF anion exchange chromatography**

Q-FF matrix was obtained from GE Healthcare. Tetraborate was added to SP-FF eluate, from Example 1 (Table 4, wash regime C), at a concentration equivalent to 100 moles borate per mole rTf (equivalent to 0.7 mM borate in the load) and as a control no borate was added. Chromatography was performed as described above using a 0.66cm $\times$ 15cm bed height (5.13 mL) column at a matrix capacity of 30 mg.mL$^{-1}$ matrix using Q-FF. Wash was carried out with 15.7 mM potassium tetraborate. Elution was carried out with 150 mM potassium tetraborate. Fractions were collected during load, wash, elution and NaCl clean and the rTf concentration estimated by RP-HPLC and the recoveries tabulated (Table 5). The flow-through (FT) was also analysed and tabulated (Table 5).

**Table 5: Effect of the absence (A) and presence (B) of borate in anion exchange chromatography load buffer on the recovery of transferrin from Q-FF anion exchange chromatography**

| A: 0 mol.mol borate in load | | | | B: 100 mol.mol borate in load | | |
|---|---|---|---|---|---|---|
| Fraction | Total (mg) | Recovery (%) | | Fraction | Total (mg) | Recovery (%) |
| Load | 151.7 | | | Load | 150.9 | |
| FT | 43.1 | 28.4 | | FT | 1.7 | 1.1 |
| Wash | 16.4 | 10.8 | | Wash | 19.4 | 12.8 |
| Elution | 75.6 | 49.8 | | Elution | 113.6 | 75.3 |
| NaCl | 12.8 | 29.7 | | NaCl | 13.3 | 8.8 |
| Mass Balance | | 119 | | Mass Balance | | 98 |

Use of borate in the load gave a higher overall recovery of transferrin in the eluate (75.3 % recovery in the presence of borate, 49.8 % recovery in the absence of borate). Also, less transferrin was present in the flow through (FT) when the load included borate (1.1 % in the presence of borate, 28.4 % in the absence of borate).

**EXAMPLE 3: Affect of presence of borate in anion exchange chromatography load buffer on the recovery of transferrin from the Capto™Q anion exchange chromatography**

Capto™Q Matrix was obtained from GE Healthcare. Potassium tetraborate was added to Capto™Q load as follows, SP-FF eluate from Example 1 (Table 4, wash regime C) was diluted 5.7 fold with a 1:1 dilution of 15.7 mM potassium tetraborate in water. Finally, sufficient water and 27% (w/v) NaOH was added to achieve a conductivity of 3.5 mS.cm$^{-1}$ and pH 8.8 (equivalent to 824 mol.mol$^{-1}$, 5.4 mM borate in the load). As a control (no borate) SP-FF eluate was conditioned with water, 27% (w/v) NaOH and 5 M NaCl to achieve pH 8.8 and a conductivity of 3.5 mS.cm$^{-1}$. Sufficient load was applied at flow rate of 0.7 mL.min$^{-1}$ onto a 0.66cm $\times$ 2cm bed height (0.68 mL) column to achieve 40 mg.mL$^{-1}$ matrix. Wash was carried out with 15.7 mM potassium tetraborate. Elution was carried out with 150 mM potassium tetraborate. Fractions were collected during load, wash, elution and NaCl clean and the rTf concentration estimated by RP-HPLC and the recoveries tabulated (Table 6). The flow-through (FT) was also analysed and tabulated (Table 6).

**Table 6: Effect of the absence (A) and presence (B) of borate in anion exchange chromatography load buffer on the recovery of transferrin from Capto™Q anion exchange chromatography**

| A: 0 mol.mol borate in load | | | | B: 825 mol.mol borate in load | | |
|---|---|---|---|---|---|---|
| Fraction | Total (mg) | Recovery (%) | | Fraction | Total (mg) | Recovery (%) |
| Load | 28.0 | | | Load | 27.0 | |
| FT | 8.8 | 31.3 | | FT | 3.1 | 11.4 |
| Wash | 2.5 | 9.0 | | Wash | 1.2 | 4.3 |
| Elution | 15.0 | 53.6 | | Elution | 21.7 | 80.6 |
| Mass Balance | | 94 | | Mass Balance | | 96 |

Use of borate in the load gave a higher overall recovery of transferrin in the eluate (80.6 % recovery in the presence of borate, 53.6 % recovery in the absence of borate). Also, less transferrin was present in the flow through (FT) when the load included borate (11.4 % in the presence of borate, 31.3 % in the absence of borate).

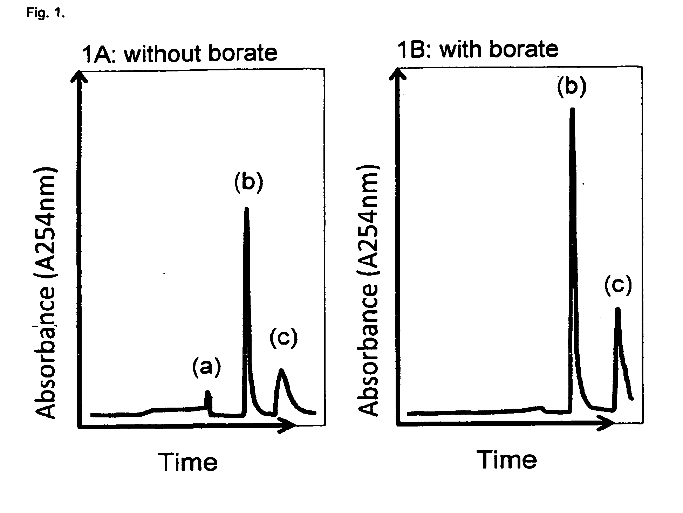

The data are also shown in Figure 1. Figure 1 shows that inclusion of borate in the load improves the binding of transferrin to the column. Figure 1A is a trace for a Capto™Q column for which tetraborate was not added to the transferrin prior to loading onto the column. Figure 1B is a trace for a Capto™Q column for which 824mol.mol$^{-1}$ tetraborate was to the transferrin prior to loading onto the column.

Peak (a) relates to transferrin present in the wash following washing of the column. Peak (b) relates to transferrin present in the eluate. Peak (c) relates to transferrin recovered from the column following washing with NaOH.

The absence of a peak (a) on Figure 1B shows that inclusion of tetraborate in the load improves the binding of transferrin to the column. The presence of peak (a) on Figure 1A shows that loading transferrin on to the column in the absence of tetraborate results in some transferrin being undesirably eluted from the column during the washing step.

### EXAMPLE 4: Low concentration wash of anionic exchange matrix

Capto™Q chromatography was performed using a 0.66 × 15cm (5.13 mL) column equilibrated in 15.7 mM potassium tetraborate. SP-FF eluate, from Example 1 (Table 4, wash regime C), was used as load material. The load was conditioned for Capto™Q chromatography by the addition 27% (w/v) NaOH to achieve a load pH of 9.0-9.2 and water to achieve a conductivity -2.6 mS.cm$^{-1}$. Finally, solid potassium tetraborate was added to achieve a final concentration of 5 mM tetraborate (725 mol.mol$^{-1}$) increasing the load conductivity to ~3.4 mS.cm$^{-1}$. A column capacity of 30 mg.mL$^{-1}$ matrix was used. Following load, the column was washed with 5 mM potassium tetraborate (10CV), 15.7 mM potassium tetraborate (11 CV) and eluted with 150 mM potassium tetraborate pH 9.4 prior to regeneration of the column with 1 M NaCl, 0.5 M NaOH and 1 hour sanitisation with 1 M NaOH.

**Table 7: Recovery of transferrin over Capto™Q chromatography using SP-FF eluate conditioned with 5 mM tetraborate in the load**

| Fraction | Buffer | Recovery (%) |
|---|---|---|
| Load | pH 9.0, 3.42 mS.cm$^{-1}$ 5 mM potassium tetraborate | 100 |
| FT | - | 1.9 |
| Wash 1 | 5 mM potassium tetraborate | 0.1 |
| Wash 2 | 15.7 mM potassium tetraborate | 0.2 |
| Elution | 150 mM potassium tetraborate | 93.2 |
| Clean 1 | 1 M NaCl + 0.5% (w/v) Polysorbate 80 | 5.5 |
| Mass Balance | | 101.2 |

The data (Table 7) show that the loading of transferrin in the presence of tetraborate, followed by and low concentration wash and a high concentration wash results in low levels of loss of transferrin during each wash step and subsequently a very high level of transferrin (93.2 % recovery) in the eluate.

### EXAMPLE 5: Mathematical modelling of low pH and salt concentration with regards removal of yeast antigen (HCP) and recovery.

The use of a low pH, high salt concentration wash regime to remove yeast antigens (YA) (*i.e.* host cell proteins (HCP)) and maintain eluate recovery on SP-FF was investigated further. A Design of Experiments (DoE) approach was employed to allow construction of a mathematical model of the effects of the two factors, pH and salt concentration, on the two responses, eluate recovery and YA clearance. A number of pH and salt concentrations in the range pH 3.66 to 5.34 and 0 mM to 6000 mM sodium chloride were studied. Load material was prepared by adjusting the pH and conductivity of culture supernatant to 5.11 and 3.57mS.cm$^{-1}$ using glacial acetic acid and water. Fifteen Atoll 5mm dia. × 2.5mm bed height MediaScout® MiniColumns (Atoll GmbH, Weingarten, Germany) containing 50μL SP-FF matrix (GE Healthcare) were equilibrated with 50mM sodium acetate pH 5.1. This load material was applied at 50μL.min$^{-1}$ to achieve a 40mg.mL$^{-1}$ matrix loading. Each column was washed first with the appropriate low pH sodium acetate buffer without NaCl (wash 1) and then with a second sodium acetate buffer of equivalent pH but containing the appropriate concentration of NaCl (wash 2) (Table 8). Each column was then eluted with 50mM sodium phosphate, 150mM sodium chloride pH 6.0 and cleaned with 1 M NaCl, 0.5% (w/w) polysorbate 80. All chromatographic fractions were collected and the transferrin concentration in each fraction was estimated by reverse phase high performance liquid chromatography (RP.HPLC). YA levels were estimated by enzyme linked immunosorbent assay (ELISA). Recoveries and YA clearance were tabulated and are shown in Table 8.

**Table 8: The effect of varying combinations of low pH and high salt concentration in wash 2 on transferrin recovery and YA clearance in the SP-FF eluate fraction.**

| Run number | Factors | | Responses | |
|---|---|---|---|---|
| | pH | NaCl Concentration (mM) | Recovery (%) | YA clearance (fold) |
| 1 | 4.00 | 5000 | 73.6 | 269 |
| 2 | 4.50 | 2525 | 76.2 | 324 |
| 3 | 3.66 | 2525 | 60.6 | 276 |
| 4 | 4.00 | 50 | 69.7 | 256 |
| 5 | 4.00 | 5000 | 68.0 | 286 |
| 6 | 4.50 | 2525 | 75.6 | 314 |
| 7 | 4.50 | 0 | 81.9 | 298 |
| 8 | 5.34 | 2525 | 26.5 | 213 |
| 9 | 5.00 | 5000 | 54.9 | 292 |
| 10 | 4.50 | 2525 | 73.6 | 345 |
| 11 | 4.50 | 6000 | 74.4 | 364 |
| 12 | 5.00 | 5000 | 21.9 | 208 |
| 13 | 5.00 | 50 | 81.8 | 356 |
| 14 | 4.00 | 50 | 56.1 | 287 |
| 15 | 5.00 | 50 | 80.8 | 383 |

Data for the recovery and YA clearance at each of the 15 wash conditions ('runs') were entered into the DoE software (Design Expert 6.0.10, Stat-Ease, Inc., Minneapolis, MN, USA) to create a mathematical model of the design space. A computer uses 'design points' (i.e. experimental data points) to generate the mathematical model of the design space. The model was fitted for each of the responses as follows

(i) Recovery:

- No transformation was required and a quadratic fit was used.
- ANOVA (analysis of variance) data indicated that the fit was significant but that the salt$^2$ term was not required.
- Therefore the salt$^2$ term was removed from the model and the data refitted to produce the following equation:

Recovery =

$$-940.50069 + (441.61018 \times p) + (0.043309 \times s) - (47.57735 \times p^2) - (0.010263 \times p \times s)$$

Where: 'p' = pH and 's' = salt concentration in mM and recovery is % recovery.

(ii) HCP (YA) Clearance:

- No transformation was required and a quadratic fit was used.
- ANOVA data indicated that a quadratic fit was not significant; therefore all terms were included for a cubic fit.
- ANOVA data indicated that the fit was now significant but that the salt$^3$ term was not required.
- Therefore the salt$^3$ term was removed from the model and the data refitted.
- ANOVA data indicated that the fit was significant but that the salt$^2$ term was not required.
- Therefore the salt$^2$ term was removed from the model and the data refitted.
- ANOVA data indicated that the fit was significant but that the pH$\times$salt$^2$ term was not required as it was aliased with the pH and pH$^3$ terms.
- Therefore the ph$\times$salt$^2$ term was removed and the data refitted to produce the following equation:

HCP (YA) clearance =

16388.65744 − (10512.30681×p) − (1.70101×s) + (2257.17601×p$^2$) + (0.78628×p×s)

− (159.06573×p$^3$) − (0.090182×p$^2$×s)

Where: 'p' = pH and 's' = salt concentration in mM and clearance is 'fold-clearance'.

Using limits of greater than or equal to 60% and greater than or equal to 275-fold ('≥' means 'greater than or equal to'), for acceptable performance of recovery and HCP (YA) clearance respectively, a graphical representation (overlay plot) of a design space was plotted (Figure 2). The areas shaded in grey (*i.e.* I, III and IV) represent those combinations of pH and salt concentration where either recovery and/or HCP (YA) clearance fall outside the acceptable performance limits; whilst the area shown in white (*i.e.* II) represents those combinations where both pH and salt concentration fall within the acceptable performance limits (i.e. greater than or equal to 60% recovery and greater than or equal to 275-fold HCP (YA) clearance).

EXAMPLE 6: Addition of potassium tetraborate to Capto Q load

Addition of potassium tetraborate to the Capto Q load material in the range 1 to 1000mol.mol was investigated. Load material was prepared by adjusting the pH and conductivity of SP-FF eluate (from Example 1) to 9.3 and 3.53mS.cm$^{-1}$ using 0.5M NaOH and water. Sufficient 0.5M potassium tetraborate was added to 30mL of this solution to achieve a load sample with a molar ratio of 1000mol borate:1mol transferrin. Individual load samples were prepared by serially diluting the 1000mol.mol solution to 500, 100, 50, 10, 5, 2.5, and 1mol.mol with the pH-adjusted, diluted solution prepared above. The conductivity of each load sample was measured and readjusted to 3.5mS.cm$^{-1}$ with water. Eight Atoll 5mm dia. × 2.5mm bed height MediaScout® MiniColumns containing 50μL Capto Q matrix (GE Healthcare) were equilibrated first with 110mM potassium tetraborate and then with 30mM potassium tetraborate. A sufficient volume of each load sample was applied at 50 μL.min$^{-1}$ to achieve a 40mg.mL$^{-1}$ matrix loading. Each column was washed first with 5mM potassium tetraborate (wash 1) and then with 30mM potassium tetraborate (wash 2) before being eluted with 110mM potassium tetraborate and cleaned with 1M NaCl, 0.5% (w/w) polysorbate 80. All chromatographic fractions were collected and the transferrin concentration estimated by reverse phase high performance liquid chromatography (RP.HPLC). Recoveries were tabulated and are shown in Table 9.

**Table 9: The effect of potassium tetraborate in Capto Q load material between 1 and 1000mol.mol on transferrin recovery in the flow through and eluate fractions.**

| Load conditions | Recovery (%) | | | | | |
|---|---|---|---|---|---|---|
| | Flow through | Wash 1 | Wash 2 | Eluate | Salt clean | Mass Balance |
| 1000mol.mol tetraborate | 0.8 | 0.1 | 1.7 | 93.7 | 5.9 | 102.2 |
| 500mol.mol tetraborate | 2.2 | 0.2 | 2.5 | 91.0 | 5.9 | 101.8 |
| 100mol.mol tetraborate | 3.4 | 0.4 | 3.1 | 78.7 | 5.6 | 91.2 |
| 50mol.mol tetraborate | 6.0 | 0.5 | 2.6 | 75.5 | 4.8 | 89.4 |
| 10mol.mol tetraborate | 17.0 | 0.9 | 1.9 | 62.2 | 4.9 | 87.0 |
| 5mol.mol tetraborate | 12.2 | 1.1 | 1.1 | 48.6 | 4.0 | 87.0 |
| 2.5mol.mol tetraborate | 18.5 | 1.1 | 1.7 | 60.5 | 4.4 | 86.2 |
| 1mol.mol tetraborate | 14.0 | 1.0 | 2.3 | 65.4 | 4.6 | 87.3 |

The data indicated that between 50 and 1000mol.mol the addition of potassium tetraborate to the load was having the desired beneficial effect and reducing the amount of transferrin lost in the flow through fraction, resulting in an increase in the amount recovered in the eluate. Between 10 and 50mol.mol there was a significant increase in the amount of transferrin lost in the flow through resulting in a reduction in the amount recovered in the eluate. In order to better define the cut-off point for the effect of potassium tetraborate addition a second experiment was performed, essentially identical to that described above, but loading at 10, 20, 30, 40 and 50mol.mol. The results of the second experiment are shown in Table 10.

**Table 10: The effect of potassium tetraborate in Capto Q load material between 10 and 50mol.mol on** transferrin recovery in the flow through and eluate fractions.

| Load conditions | Recovery (%) | | | | | |
|---|---|---|---|---|---|---|
| | Flow through | Wash 1 | Wash 2 | Eluate | Salt clean | Mass Balance |
| 50mol.mol tetraborate | 0.5 | 0.1 | 1.2 | 91.1 | 7.9 | 100.7 |
| 40mol.mol tetraborate | 7.3 | 0.4 | 3.6 | 78.8 | 5.7 | 95.8 |
| 30mol.mol tetraborate | 6.9 | 0.5 | 3.1 | 78.7 | 5.9 | 95.0 |
| 20mol.mol tetraborate | 7.5 | 0.5 | 3.4 | 91.0 | 5.8 | 108.2 |
| 10mol.mol tetraborate | 18.8 | 0.9 | 2.1 | 65.7 | 7.2 | 94.7 |

This second set of data indicated that between 20 and 50mol.mol the addition of potassium tetraborate was having the desired beneficial effect and reducing the amount of transferrin lost in the flow through fraction, resulting in an increase in the amount recovered in the eluate. Below 10mol.mol there was a significant increase in the amount of transferrin lost in the flow through resulting in a reduction in the amount recovered in the eluate.

In conclusion the addition of potassium tetraborate to the Capto Q load in the range 20 to 1000mol.mol has a beneficial effect by reducing transferrin losses in the flow through with a resultant increase in the amount of transferrin recovered in the eluate.

## EXAMPLE 7: Addition of potassium tetraborate to Capto Q wash 1

Addition of potassium tetraborate to the Capto Q wash 1 in the range 0 to 30mM was investigated. Load material was prepared by adjusting the pH and conductivity of SP-FF eluate (from Example 1) to 9.3 and 3.53mS.cm$^{-1}$ using 0.5M NaOH and water. Sufficient 0.5M potassium tetraborate was added to 50mL of this solution to achieve a load sample with a molar ratio of 270mol borate:1mol transferrin. The conductivity of the load sample was measured and readjusted to 3.5mS.cm$^{-1}$ with water. Nine Atoll 5mm dia. $\times$ 2.5mm bed height MediaScout® MiniColumns containing 50$\mu$L Capto Q matrix (GE Healthcare) were equilibrated first with 110mM potassium tetraborate then with 30mM potassium tetraborate. A sufficient volume of each load sample was applied at 50 $\mu$L.min$^{-1}$ to achieve a 40mg.mL$^{-1}$ matrix loading. Each column was washed first with potassium tetraborate at either 0, 0.1, 0.5, 1, 2, 5, 10, 20 or 30mM (wash 1) and then with 30mM potassium tetraborate (wash 2) before being eluted with 110mM potassium tetraborate and cleaned with 1M NaCl, 0.5% (w/w) polysorbate 80. All chromatographic fractions were collected and the transferrin concentration estimated by RP.HPLC. Recoveries were tabulated and are shown in Table 11.

**Table 11: The effect of potassium tetraborate in Capto Q wash 1 between 0 and 30mM on transferrin recovery in the wash 1 and wash 2 fractions.**

| Wash 1 conditions | Recovery (%) | | | | | |
|---|---|---|---|---|---|---|
| | Flow through | Wash 1 | Wash 2 | Eluate | Salt clean | Mass Balance |
| 30mM tetraborate | 4.5 | 9.5 | 1.2 | 76.1 | 5.3 | 96.6 |

(continued)

| Wash 1 conditions | Recovery (%) | | | | | |
|---|---|---|---|---|---|---|
| | Flow through | Wash 1 | Wash 2 | Eluate | Salt clean | Mass Balance |
| 20mM tetraborate | 4.0 | 3.1 | 0.9 | 79.5 | 7.4 | 94.9 |
| 10mM tetraborate | 2.6 | 0.3 | 1.5 | 83.8 | 7.2 | 95.4 |
| 5mM tetraborate | 3.4 | 0.1 | 2.2 | 79.5 | 10.9 | 96.2 |
| 2mM tetraborate | 5.6 | 0.4 | 2.7 | 80.4 | 6.2 | 95.3 |
| 1mM tetraborate | 5.6 | 0.5 | 3.5 | 81.0 | 5.1 | 95.7 |
| 0.5mM tetraborate | 6.5 | 0.7 | 4.2 | 80.3 | 5.0 | 96.7 |
| 0.1mM tetraborate | 6.4 | 0.4 | 6.2 | 87.5 | 5.9 | 106.4 |
| 0mM tetraborate | 5.1 | 0.1 | 7.2 | 75.9 | 7.6 | 96.0 |

The data indicated that a concentration of between 0.5 and 20mM potassium tetraborate in wash 1 was having the desired beneficial effect *i.e.* reducing the amount of transferrin lost in wash 2. Above 20mM there was a significant increase in the amount of transferrin lost in wash 1 whilst between 0 and 0.1mM there was an unacceptable increase in the amount of transferrin lost in wash 2.

### EXAMPLE 8: Addition of sodium tetraborate to Capto Q load

Addition of sodium tetraborate to the Capto Q load material was investigated. Load material was prepared by adjusting the pH and conductivity of SP-FF eluate to 9.4 and 3.66mS.cm$^{-1}$ using 0.5M NaOH and water. Sufficient 110mM sodium tetraborate was added to half of this solution to achieve a load sample with a molar ratio of 270mol borate:1mol transferrin. The conductivity of the load sample was measured and readjusted to 3.61 mS.cm$^{-1}$ with water. Two Atoll 5mm dia. $\times$ 2.5mm bed height MediaScout$^®$ MiniColumns containing 50$\mu$L Capto Q matrix (GE Healthcare) were equilibrated first with 110mM sodium tetraborate then with 30mM sodium tetraborate. Sufficient of the 270mol.mol load sample and a control of pH adjusted, diluted material containing no sodium tetraborate were applied at 50$\mu$L.min$^{-1}$ to achieve a 40mg.ml$^{-1}$ matrix loading. Each column was washed first with 5mM sodium tetraborate (wash 1) and then with 30mM sodium tetraborate (wash 2) before being eluted with 110mM sodium tetraborate and cleaned with 1 M NaCl, 0.5% (w/w) polysorbate 80. All chromatographic fractions were collected and the transferrin concentration estimated by reverse phase high performance liquid chromatography (RP.HPLC). Recoveries were tabulated and are shown in Table 12.

**Table 12: The effect of sodium tetraborate in Capto Q load at 270mol.mol on transferrin recovery in the flow through and eluate fractions.**

| Load conditions | Recovery (%) | | | | | |
|---|---|---|---|---|---|---|
| | Flow through | Wash 1 | Wash 2 | Eluate | Salt clean | Mass Balance |
| 0mol.mol tetraborate | 16.9 | 0.9 | 3.2 | 63.2 | 5.2 | 89.5 |
| 270mol.mol tetraborate | 5.1 | 0.2 | 2.7 | 75.2 | 4.9 | 88.1 |

The data indicated that the addition of sodium tetraborate to the load at 270mol.mol was having the desired beneficial effect *i.e.* reducing the amount of transferrin lost in the flow through fraction, resulting in an increase in the amount recovered in the eluate.

Claims

1. A process for purifying a transferrin solution, the process comprising:

    a) adding tetraborate to a relatively impure solution of transferrin to provide a solution of transferrin and tetrab-

38

orate;
b) applying the solution of transferrin and tetraborate to an anionic chromatographic material for which the transferrin has no specific binding activity such that the transferrin binds to the material;
c) washing the chromatographic material to which the transferrin is bound;
d) optionally, further washing the chromatographic material to which the transferrin is bound; and
e) optionally, recovering the transferrin from the anionic chromatographic material.

2. A process according to claim 1 wherein: in step (a), the solution of transferrin and tetraborate is at a ratio of at least 20 mole tetraborate to 1 mole transferrin.

3. A process according to claim 1 or 2 in which the washing of step (c) is carried out using a washing solution having a lower tetraborate concentration than the washing solution used for the washing of step (d).

4. A process according to any preceding claim in which the washing of step (c) is carried out using a tetraborate solution of from 0.1 mM to 20 mM.

5. A process according to any preceding claim in which the washing of step (d) is carried out using a tetraborate solution of from 5 mM to 60 mM.

6. A process according to any preceding claim comprising:

a) adding tetraborate to a relatively impure solution of transferrin to provide a solution of transferrin and tetraborate at a ratio of at least 20 mole tetraborate to 1 mole transferrin;
b) applying the solution of transferrin and tetraborate to an anionic chromatographic material for which the transferrin has no specific binding affinity such that the transferrin binds to the material;
c) washing the chromatographic material with a tetraborate solution of from 0.1 mM to 20 mM;
d) subsequently washing the chromatographic material with a tetraborate solution of at least 30 mM; and
e) optionally, recovering the transferrin from the anionic chromatographic material.

7. A process for purifying a transferrin solution, the process comprising an anionic chromatographic step and a cationic chromatographic step, in which:

the cationic chromatographic step comprises:

a) applying a relatively impure solution of a transferrin to a cationic chromatographic material for which the transferrin has no specific affinity such that the transferrin binds to the material;
b) washing the cationic chromatographic material with a wash buffer having a pH of from 4 to 5 and being substantially free of salts other than the salt which provides the buffering effect;
c) subsequently washing the cationic chromatographic material with a wash buffer having a pH from 4 to 5 and a salt concentration of from 50 mM to 5 M; and
d) recovering the transferrin from the cationic chromatographic material; and

the anionic chromatographic step comprises:

a) adding tetraborate to transferrin obtained from step (d) of the cationic chromatographic step to provide a solution of transferrin and tetraborate;
b) applying the solution of transferrin and tetraborate to an anionic chromatographic material for which the transferrin has no specific activity such that the transferrin binds to the material;
c) washing the chromatographic material to which the transferrin is bound;
d) optionally further washing the chromatographic material to which the transferrin is bound; and
e) optionally, recovering the transferrin from the anionic chromatographic material.

8. A process according to any of claims 1 to 7 wherein the tetraborate solution used in the washing of step (c) of the anionic chromatographic step is from 2.5 mM to 7.5 mM.

9. A process according to claim 8 wherein the tetraborate solution used in the washing of step (c) of the anionic chromatographic step is 5 mM.

10. A process according to any of claims 1 to 9 wherein the tetraborate solution used in the washing of step (d) of the anionic chromatographic step is from 20 mM to 40 mM.

11. A process according to claim 10 wherein the tetraborate solution used in the washing of step (d) of the anionic chromatographic step is 30 mM.

12. A process according to any of claims 1 to 11 wherein the tetraborate is potassium tetraborate or sodium tetraborate.

13. A process according to any of claims 1 to 12 wherein the anionic chromatographic material comprises a functional ligand selected from: a diethylaminoethyl (DEAE) group and a quaternary amine group.

14. A process according to any of claims 7 to 13 in which the pH of the wash buffer of step (b) of the cationic chromatographic step is from 4.3 to 4.7.

15. A process according to claim 14 in which the pH of the wash buffer of step (b) is 4.5.

16. A process according to any of claims 7 to 15 in which the wash buffer of step (b) of the cationic chromatographic step has a salt concentration of less than 50 mM.

17. A process according to claim 16 in which the wash buffer of step (b) does not comprise salt other than the salt which provides the buffering effect.

18. A process according to any of claims 7 to 17 in which the wash of step (c) of the cationic chromatographic step:

  (i) lies within a design space defined by both of the following equations:

$$\text{(I)} \quad \text{Recovery} = -940.50069 + (441.61018 \times p) + (0.043309 \times s) - (47.57735 \times p2) - (0.010263 \times p \times s)$$

$$\text{(II)} \quad \text{Host cell protein clearance} = 16388.65744 - (10512.30681 \times p) - (1.70101 \times s) + (2257.17601 \times p2) + (0.78628 \times p \times s) - (159.06573 \times p3) - (0.090182 \times p2 \times s)$$

  where p = pH, s = salt concentration in mM, Recovery is greater than or equal to 60% and YA clearance is greater than or equal to 275-fold; and
  (ii) has a pH of from 4 to 5 and a salt concentration of from 50 to 5000 mM.

19. A process according to any of claims 7 to 18 in which the pH of the wash buffer of step (c) of the cationic chromatographic step is from 4.3 to 4.7.

20. A process according to claim 19 in which the pH of the wash buffer of step (c) is 4.5.

21. A process according to any of claims 7 to 20 in which the wash buffer of step (c) of the cationic chromatographic step has a salt concentration of from 250 mM to 2.5 M.

22. A process according to claim 21 in which the wash buffer of step (c) has a salt concentration of 2 M.

23. A process according to any of claims 7 to 22 in which the salt of step (b) and/or step (c) of the cationic chromatographic step is NaCl.

24. A process according to any of claims 7 to 23 in which the cationic chromatographic material comprises a functional ligand comprising a sulphopropyl group.

25. A process according to any of claims 7 to 24 in which the cationic chromatographic step further comprises a third wash prior to recovery of the transferrin.

26. A process according to claim 25 in which the third wash is carried out using a wash buffer comprising 50 mM sodium acetate and at pH 5.1.

**Patentansprüche**

1.  Verfahren zum Reinigen einer Transferrinlösung, wobei das Verfahren umfasst:

    a) Hinzufügen von Tetraborat zu einer relativ unsauberen Lösung von Transferrin, um eine Lösung von Transferrin und Tetraborat bereitzustellen;
    b) Auftragen der Lösung von Transferrin und Tetraborat auf ein anionisches Chromatographiematerial, für das das Transferrin keine spezifische Bindungsaktivität aufweist, so dass das Transferrin an das Material bindet;
    c) Waschen des Chromatographiematerials, an das das Transferrin gebunden ist;
    d) gegebenenfalls weiteres Waschen des Chromatographiematerials, an das das Transferrin gebunden ist; und
    e) gegebenenfalls Gewinnen des Transferrins von dem anionischen Chromatographiematerial.

2.  Verfahren nach Anspruch 1, wobei: in Schritt (a) die Lösung von Transferrin und Tetraborat in einem Verhältnis von mindestens 20 mol Tetraborat zu 1 mol Transferrin vorliegt.

3.  Verfahren nach Anspruch 1 oder 2, in dem das Waschen von Schritt (c) unter Verwendung einer Waschlösung mit einer niedrigeren Tetraboratkonzentration als die in der für das Waschen von Schritt (d) verwendeten Waschlösung durchgeführt wird.

4.  Verfahren nach einem beliebigen vorhergehenden Anspruch, in dem das Waschen von Schritt (c) unter Verwendung einer Tetraboratlösung von 0,1 mM bis 20 mM durchgeführt wird.

5.  Verfahren nach einem beliebigen vorhergehenden Anspruch, in dem das Waschen von Schritt (d) unter Verwendung einer Tetraboratlösung von 5 mM bis 60 mM durchgeführt wird.

6.  Verfahren nach einem beliebigen vorhergehenden Anspruch, umfassend:

    a) Hinzufügen von Tetraborat zu einer relativ unsauberen Lösung von Transferrin, um eine Lösung von Transferrin und Tetraborat in einem Verhältnis von mindestens 20 mol Tetraborat zu 1 mol Transferrin bereitzustellen;
    b) Auftragen der Lösung von Transferrin und Tetraborat auf ein anionisches Chromatographiematerial, für das das Transferrin keine spezifische Bindungsaffinität aufweist, so dass das Transferrin an das Material bindet;
    c) Waschen des Chromatographiematerials mit einer Tetraboratlösung von 0,1 mM bis 20 mM;
    d) anschließend Waschen des Chromatographiematerials mit einer Tetraboratlösung von mindestens 30 mM; und
    e) gegebenenfalls Gewinnen des Transferrins von dem anionischen Chromatographiematerial.

7.  Verfahren zum Reinigen einer Transferrinlösung, wobei das Verfahren einen anionischen Chromatographieschritt und einen kationischen Chromatographieschritt umfasst, in dem:

    der kationische Chromatographieschritt umfasst:

    a) Auftragen einer relativ unsauberen Lösung eines Transferrins auf ein kationisches Chromatographiematerial, für das das Transferrin keine spezifische Affinität aufweist, so dass das Transferrin an das Material bindet;
    b) Waschen des kationischen Chromatographiematerials mit einem Waschpuffer, der einen pH von 4 bis 5 aufweist, und außer dem Salz, das die Pufferwirkung bereitstellt, im Wesentlichen frei von Salzen ist;
    c) Anschließend Waschen des kationischen Chromatographiematerials mit einem Waschpuffer mit einem pH von 4 bis 5 und einer Salzkonzentration von 50 mM bis 5 M; und
    d) Gewinnen des Transferrins von dem kationischen Chromatographiematerial; und

    der anionische Chromatographieschritt umfasst:

    a) Hinzufügen von Tetraborat zu aus Schritt (d) des kationischen Chromatographieschritts erhaltenem Transferrin, um eine Lösung von Transferrin und Tetraborat bereitzustellen;
    b) Auftragen der Lösung von Transferrin und Tetraborat auf ein anionisches Chromatographiematerial, für das das Transferrin keine spezifische Aktivität aufweist, so dass das Transferrin an das Material bindet;
c) Waschen des Chromatographiematerials, an das das Transferrin gebunden ist;
d) gegebenenfalls weiteres Waschen des Chromatographiematerials, an das das Transferrin gebunden ist; und
e) gegebenenfalls Gewinnen des Transferrins von dem anionischen Chromatographiematerial.

8. Verfahren nach einem beliebigen der Ansprüche 1 bis 7, wobei die in dem Waschen von Schritt (c) des anionischen Chromatographieschritts verwendete Tetraboratlösung von 2,5 mM bis 7,5 mM ist.

9. Verfahren nach Anspruch 8, wobei die in dem Waschen von Schritt (c) des anionischen Chromatographieschritts verwendete Tetraboratlösung 5 mM ist.

10. Verfahren nach einem beliebigen der Ansprüche 1 bis 9, wobei die in dem Waschen von Schritt (d) des anionischen Chromatographieschritts verwendete Tetraboratlösung von 20 mM bis 40 mM ist.

11. Verfahren nach Anspruch 10, wobei die in dem Waschen von Schritt (d) des anionischen Chromatographieschritts verwendete Tetraboratlösung 30 mM ist.

12. Verfahren nach einem beliebigen der Ansprüche 1 bis 11, wobei das Tetraborat Kaliumtetraborat oder Natriumtetraborat ist.

13. Verfahren nach einem beliebigen der Ansprüche 1 bis 12, wobei das anionische Chromatographiematerial einen funktionellen Liganden umfasst, ausgewählt aus: einer Diethylaminoethyl(DEAE)-Gruppe und einer quaternären Amingruppe.

14. Verfahren nach einem beliebigen der Ansprüche 7 bis 13, in dem der pH des Waschpuffers von Schritt (b) des kationischen Chromatographieschritts von 4,3 bis 4,7 beträgt.

15. Verfahren nach Anspruch 14, in dem der pH des Waschpuffers von Schritt (b) 4,5 beträgt.

16. Verfahren nach einem beliebigen der Ansprüche 7 bis 15, in dem der Waschpuffer von Schritt (b) des kationischen Chromatographieschritts eine Salzkonzentration von weniger als 50 mM aufweist.

17. Verfahren nach Anspruch 16, in dem der Waschpuffer von Schritt (b) außer dem Salz, das die Pufferwirkung bereitstellt, kein weiteres Salz umfasst.

18. Verfahren nach einem beliebigen der Ansprüche 7 bis 17, in dem das Waschen von Schritt (c) des kationischen Chromatographieschritts:

(i) innerhalb eines Designraums liegt, definiert durch beide der folgenden Gleichungen:

$$(I) \quad \text{Gewinnung} = -940{,}50069 + (441{,}61018xp) + (0{,}043309xs) - (47{,}57735xp2) - (0{,}010263xpxs)$$

$$(II) \quad \text{Wirtszellprotein-Clearance} = 16388{,}65744 - (10512{,}30681xp) - (1{,}70101xs) + (2257{,}17601xp2) + (0{,}78628xpxs) - (159{,}06573xp3) - (0{,}090182xp2xs)$$

wobei p = pH, s = Salzkonzentration in mM, Gewinnung ist größer als oder gleich 60% und YA-Clearance ist größer oder gleich 275-fach; und
(ii) einen pH von 4 bis 5 und eine Salzkonzentration von 50 bis 5000 mM aufweist.

19. Verfahren nach einem beliebigen der Ansprüche 7 bis 18, in dem der pH des Waschpuffers von Schritt (c) des kationischen Chromatographieschritts von 4,3 bis 4,7 beträgt.

20. Verfahren nach Anspruch 19, in dem der pH des Waschpuffers von Schritt (c) 4,5 beträgt.

**21.** Verfahren nach einem beliebigen der Ansprüche 7 bis 20, in dem der Waschpuffer von Schritt (c) des kationischen Chromatographieschritts eine Salzkonzentration von 250 mM bis 2,5 M aufweist.

**22.** Verfahren nach Anspruch 21, in dem der Waschpuffer von Schritt (c) eine Salzkonzentration von 2 M aufweist.

**23.** Verfahren nach einem beliebigen der Ansprüche 7 bis 22, in dem das Salz von Schritt (b) und/oder Schritt (c) des kationischen Chromatographieschritts NaCl ist.

**24.** Verfahren nach einem beliebigen der Ansprüche 7 bis 23, in dem das kationische Chromatographiematerial einen funktionellen Liganden umfasst, der eine Sulfopropylgruppe umfasst.

**25.** Verfahren nach einem beliebigen der Ansprüche 7 bis 24, in dem der kationische Chromatographieschritt des Weiteren eine dritte Wäsche vor der Gewinnung des Transferrins umfasst.

**26.** Verfahren nach Anspruch 25, in dem die dritte Wäsche unter Verwendung eines Waschpuffers durchgeführt wird, der 50 mM Natriumacetat und pH 5,1 umfasst.

**Revendications**

**1.** Procédé de purification d'une solution de transferrine, le procédé comprenant :

a) l'ajout de tétraborate à une solution relativement impure de transferrine pour obtenir une solution de transferrine et tétraborate ;
b) l'application de la solution de transferrine et tétraborate à un matériau chromatographique anionique pour lequel la transferrine n'a pas d'activité de liaison spécifique pour que la transferrine se lie au matériau ;
c) le lavage du matériau chromatographique auquel la transferrine est liée ;
d) éventuellement, le lavage supplémentaire du matériau chromatographique auquel la transferrine est liée ; et
e) éventuellement, la récupération de la transferrine à partir du matériau chromatographique anionique.

**2.** Procédé selon la revendication 1 dans lequel : dans l'étape (a), la solution de transferrine et tétraborate est à un rapport d'au moins 20 moles de tétraborate pour 1 mole de transferrine.

**3.** Procédé selon les revendications 1 ou 2 dans lequel le lavage de l'étape (c) est effectué à l'aide d'une solution de lavage ayant une concentration de tétraborate plus basse que la solution de lavage utilisée pour le lavage de l'étape (d).

**4.** Procédé selon l'une quelconque des revendications précédentes dans lequel le lavage de l'étape (c) est effectué à l'aide d'une solution de tétraborate à 0,1-20 mM.

**5.** Procédé selon l'une quelconque des revendications précédentes dans lequel le lavage de l'étape (d) est effectué à l'aide d'une solution de tétraborate à 5-60 mM.

**6.** Procédé selon l'une quelconque des revendications précédentes comprenant :

a) l'ajout de tétraborate à une solution relativement impure de transferrine pour obtenir une solution de transferrine et tétraborate à un rapport d'au moins 20 moles de tétraborate pour 1 mole de transferrine ;
b) l'application de la solution de transferrine et tétraborate à un matériau chromatographique anionique pour lequel la transferrine n'a pas d'affinité de liaison spécifique pour que la transferrine se lie au matériau ;
c) le lavage du matériau chromatographique avec une solution de tétraborate à 0,1-20 mM ;
d) ultérieurement, le lavage du matériau chromatographique avec une solution de tétraborate à au moins 30 mM ; et
e) éventuellement, la récupération de la transferrine à partir du matériau chromatographique anionique.

**7.** Procédé de purification d'une solution de transferrine, le procédé comprenant une étape chromatographique anionique et une étape chromatographique cationique, dans lequel :

l'étape chromatographique cationique comprend :

EP 2 393 834 B1

a) l'application d'une solution relativement impure de transferrine à un matériau chromatographique cationique pour lequel la transferrine n'a pas d'affinité spécifique pour que la transferrine se lie au matériau ;
b) le lavage du matériau chromatographique cationique avec un tampon de lavage ayant un pH de 4 à 5 et étant essentiellement dépourvu de sels autres que le sel qui fournit l'effet de tamponnage ;
c) ultérieurement, le lavage du matériau chromatographique cationique avec un tampon de lavage ayant un pH de 4 à 5 et une concentration de sel de 50 mM à 5 M ; et
d) la récupération de la transferrine à partir du matériau chromatographique cationique ; et

l'étape chromatographique anionique comprend :

a) l'ajout de tétraborate à la transferrine obtenue à l'issue de l'étape (d) de l'étape chromatographique cationique pour obtenir une solution de transferrine et tétraborate ;
b) l'application de la solution de transferrine et tétraborate à un matériau chromatographique anionique pour lequel la transferrine n'a pas d'activité spécifique pour que la transferrine se lie au matériau ;
c) le lavage du matériau chromatographique auquel la transferrine est liée ;
d) éventuellement, le lavage supplémentaire du matériau chromatographique auquel la transferrine est liée ; et
e) éventuellement, la récupération de la transferrine à partir du matériau chromatographique anionique.

8. Procédé selon l'une quelconque des revendications 1 à 7, dans lequel la solution de tétraborate utilisée dans le lavage de l'étape (c) de l'étape chromatographique anionique est à 2,5-7,5 mM.

9. Procédé selon la revendication 8, dans lequel la solution de tétraborate utilisée dans le lavage de l'étape (c) de l'étape chromatographique anionique est à 5 mM.

10. Procédé selon l'une quelconque des revendications 1 à 9 dans lequel la solution de tétraborate utilisée dans le lavage de l'étape (d) de l'étape chromatographique anionique est à 20-40 mM.

11. Procédé selon la revendication 10 dans lequel la solution de tétraborate utilisée dans le lavage de l'étape (d) de l'étape chromatographique anionique est à 30 mM.

12. Procédé selon l'une quelconque des revendications 1 à 11 dans lequel le tétraborate est le tétraborate de potassium ou le tétraborate de sodium.

13. Procédé selon l'une quelconque des revendications 1 à 12 dans lequel le matériau chromatographique anionique comprend un ligand fonctionnel choisi parmi : un groupe diéthylaminoéthyle (DEAE) et un groupe amine quaternaire.

14. Procédé selon l'une quelconque des revendications 7 à 13 dans lequel le pH du tampon de lavage de l'étape (b) de l'étape chromatographique cationique est de 4,3 à 4,7.

15. Procédé selon la revendication 14 dans lequel le pH du tampon de lavage de l'étape (b) est de 4,5.

16. Procédé selon l'une quelconque des revendications 7 à 15 dans lequel le tampon de lavage de l'étape (b) de l'étape chromatographique cationique a une concentration de sel inférieure à 50 mM.

17. Procédé selon la revendication 16 dans lequel le tampon de lavage de l'étape (b) ne comprend pas de sel autre que le sel qui fournit l'effet de tamponnage.

18. Procédé selon l'une quelconque des revendications 7 à 17 dans lequel le lavage de l'étape (c) de l'étape chromatographique cationique :

(i) s'inscrit dans un espace de conception défini par les deux équations suivantes :

$$(I)\ \text{Récupération} = -940{,}50069 + (441{,}61018xp) + (0{,}043309xs) - (47{,}57735xp2)$$
$$- (0{,}010263xpxs)$$

$$\text{(II) Clairance des protéines de la cellule hôte} = 16388,65744 - (10512,30681xp) - (1,70101xs) + (2257,17601xp2) + (0,78628xpxs) - (159,06573xp3) - (0,090182xp2xs)$$

où p = pH, s = concentration de sel en mM, la Récupération est supérieure ou égale à 60 % et la Clairance YA est supérieure ou égale à 275 fois ; et
(ii) a un pH de 4 à 5 et une concentration de sel de 50 à 5000 mM.

19. Procédé selon l'une quelconque des revendications 7 à 18 dans lequel le pH du tampon de lavage de l'étape (c) de l'étape chromatographique cationique est de 4,3 à 4,7.

20. Procédé selon la revendication 19 dans lequel le pH du tampon de lavage de l'étape (c) est de 4,5.

21. Procédé selon l'une quelconque des revendications 7 à 20 dans lequel le tampon de lavage de l'étape (c) de l'étape chromatographique cationique a une concentration de sel de 250 mM à 2,5 M.

22. Procédé selon la revendication 21 dans lequel le tampon de lavage de l'étape (c) a une concentration de sel de 2 M.

23. Procédé selon l'une quelconque des revendications 7 à 22 dans lequel le sel de l'étape (b) et/ou de l'étape (c) de l'étape chromatographique cationique est NaCl.

24. Procédé selon l'une quelconque des revendications 7 à 23 dans lequel le matériau chromatographique cationique comprend un ligand fonctionnel comprenant un groupe sulfopropyle.

25. Procédé selon l'une quelconque des revendications 7 à 24 dans lequel l'étape chromatographique cationique comprend en outre un troisième lavage avant la récupération de la transferrine.

26. Procédé selon la revendication 25 dans lequel le troisième lavage est effectué à l'aide d'un tampon de lavage comprenant 50 mM d'acétate de sodium et à un pH de 5,1.

Fig. 1.

1A: without borate

1B: with borate

Fig. 2

● **Design Points**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6251860 B
- US 5744586 A
- US 5041537 A
- EP 2008060482 W
- US 5986067 A
- WO 2008152140 A
- US 20030221201 A
- US 20040023334 A
- EP 238023 A
- US 5364770 A
- US 5578463 A
- EP 184438 A
- EP 284603 A
- WO 2000056900 A
- WO 9614413 A
- WO 9404687 A
- WO 9533833 A
- WO 9523857 A
- EP 258067 A
- EP 431880 A
- EP 60057 A
- EP 366400 A
- WO 9001063 A
- EP 424117 A
- EP 286424 A
- WO 2005061719 A
- EP 09152332 A

### Non-patent literature cited in the description

- **VAN CAMPENHOUT et al.** *Free Radic. Res.,* 2003, vol. 37, 1069-1077
- **RIVAT et al.** *Journal of Chromatography,* 1992, vol. 576, 71-77
- Liquid column chromatography: a survey of modern techniques and applications. Elsevier, 1975, 344-351
- Protein liquid chromatography. Elsevier, 2000, 18-21
- Liquid column chromatography: a survey of modem techniques and applications. Elsevier, 1975, 344-351
- **LAMBERT et al.** *Comparative Biochemistry and Physiology,* 2005, vol. 142, 129-141
- **TESTA.** *Proteins of iron metabolism,* 2002
- Iron carriers and iron proteins. **HARRIS ; AISEN.** Physical Bioinorganic Chemistry. VCH, 1991, vol. 5
- **MASON et al.** *Biochemistry,* 1993, vol. 32, 5472
- **MASON et al.** *Biochem. J.,* 1998, vol. 330, 35
- **MASON et al.** *Protein Expr. Purif.,* 1996, vol. 8, 119
- **MASON et al.** *Protein Expr. Purif.,* 1991, vol. 2, 214
- **SHIN et al.** *Proc. Natl. Acad. Sci. USA,* 1995, vol. 92, 2820
- **ALI et al.** *J. Biol. Chem.,* 1999, vol. 274, 24066
- **MASON et al.** *Biochemistry,* 2002, vol. 41, 9448
- Protein Formulation and Delivery. Marcel Dekker Inc, 2000
- Rational Design of Stable Protein Formulations - Theory and Practice. Pharmaceutical Biotechnology. Kluwer Academic/Plenum Publishers, 2002, vol. 13
- **YAZDI ; MURPHY.** *Cancer Research,* 1994, vol. 54, 6387-6394
- **WIDERA et al.** *Pharmaceutical Research,* 2003, vol. 20, 1231-1238
- **LEE et al.** *Arch. Pharm. Res.,* 2005, vol. 28, 722-729
- **SANKER.** *Genetic Eng. News,* 2004, vol. 24, 22-28
- **SCHMIDT.** *Appl. Microbiol. Biotechnol.,* 2004, vol. 65, 363-372
- **MASON et al.** *Protein Expr. Purif.,* 2004, vol. 36, 318-326
- **MASON et al.** *Biochemistry,* 2002, vol. 41, 9448-9454
- **LIM et al.** *Biotechnol. Prog.,* 2004, vol. 20, 1192-1197
- **DYCK et al.** *Trends in Biotechnology,* 2003, vol. 21, 394-399
- **MA et al.** *Nature Reviews Genetics,* 2003, vol. 4, 794-805
- **STEINLEIN ; IKEDA.** *Enzyme Microb. Technol.,* 1993, vol. 15, 193-199
- **NAVALAINEN et al.** *Trends in Biotechnology,* 2005, vol. 23, 468-473

- **LIM et al.** *Biotechnol Prog.,* 2004, vol. 20, 1192-1197
- **MASON et al.** *Protein Expr. Purif.,* 2001, vol. 23, 142-150
- **MASON et al.** *Biochemistry,* 1993, vol. 32, 5472-5479
- **SARGENT et al.** *Biometals,* 2006, vol. 19, 513-519
- **MASON et al.** *Biochem. J,* 1997, vol. 326, 77-85
- **MASON et al.** *Protein Expr. Purif.,* 1996, vol. 8, 119-125
- **STEINLEIN et al.** *Protein Expr. Purif.,* 1995, vol. 6, 619-624
- **MAYSON et al.** *Biotechnol. Bioeng.,* 2003, vol. 81, 291-298
- **DUFFY et al.** Inhibition of protein mannosyltransferase 1 (PMT1) activity in the pathogenic yeast Candida albicans. *International Conference on Molecular Mechanisms of Fungal Cell Wall Biogenesis,* 26 August 2001
- **NOTHWEHR ; GORDON.** *Bioessays,* 1990, vol. 12, 479-484
- **GIERASCH.** *Biochemistry,* 1989, vol. 28, 923-930
- **SMITH et al.** *Science,* 1985, vol. 229, 1219-1224
- **IRIE et al.** *Gene,* 1991, vol. 108 (1), 139-144
- **IRIE et al.** *Mol. Gen. Genet.,* 1991, vol. 225 (2), 257-265
- **BOTSTEIN, D. et al.** *Gene,* 1979, vol. 8, 17-24
- **GIETZ, R.D. ; SUGINO. A.** *Gene,* 1988, vol. 74, 527-534

**SEQUENCE LISTING**

<110> Novozymes Biopharma UK Limited

<120> Purification Process

<130> 11248.000-EP

<150> EP09152332
<151> 2009-02-06

<160> 4

<170> PatentIn version 3.5

<210> 1
<211> 679
<212> PRT
<213> Homo sapiens

<220>
<221> MISC_FEATURE
<222> (1)..(679)
<223> Homo sapiens transferrin

<400> 1

Val Pro Asp Lys Thr Val Arg Trp Cys Ala Val Ser Glu His Glu Ala
1              5              10              15

Thr Lys Cys Gln Ser Phe Arg Asp His Met Lys Ser Val Ile Pro Ser
        20              25              30

Asp Gly Pro Ser Val Ala Cys Val Lys Lys Ala Ser Tyr Leu Asp Cys
        35              40              45

Ile Arg Ala Ile Ala Ala Asn Glu Ala Asp Ala Val Thr Leu Asp Ala
    50              55              60

Gly Leu Val Tyr Asp Ala Tyr Leu Ala Pro Asn Asn Leu Lys Pro Val
65              70              75              80

Val Ala Glu Phe Tyr Gly Ser Lys Glu Asp Pro Gln Thr Phe Tyr Tyr
            85              90              95

Ala Val Ala Val Val Lys Lys Asp Ser Gly Phe Gln Met Asn Gln Leu
        100              105              110

Arg Gly Lys Lys Ser Cys His Thr Gly Leu Gly Arg Ser Ala Gly Trp
        115              120              125

25

Asn Ile Pro Ile Gly Leu Leu Tyr Cys Asp Leu Pro Glu Pro Arg Lys
    130             135         140

Pro Leu Glu Lys Ala Val Ala Asn Phe Phe Ser Gly Ser Cys Ala Pro
    145         150             155                 160

Cys Ala Asp Gly Thr Asp Phe Pro Gln Leu Cys Gln Leu Cys Pro Gly
            165             170             175

Cys Gly Cys Ser Thr Leu Asn Gln Tyr Phe Gly Tyr Ser Gly Ala Phe
            180             185             190

Lys Cys Leu Lys Asp Gly Ala Gly Asp Val Ala Phe Val Lys His Ser
        195         200             205

Thr Ile Phe Glu Asn Leu Ala Asn Lys Ala Asp Arg Asp Gln Tyr Glu
    210             215             220

Leu Leu Cys Leu Asp Asn Thr Arg Lys Pro Val Asp Glu Tyr Lys Asp
225             230             235             240

Cys His Leu Ala Gln Val Pro Ser His Thr Val Val Ala Arg Ser Met
            245             250             255

Gly Gly Lys Glu Asp Leu Ile Trp Glu Leu Leu Asn Gln Ala Gln Glu
        260             265             270

His Phe Gly Lys Asp Lys Ser Lys Glu Phe Gln Leu Phe Ser Ser Pro
    275             280             285

His Gly Lys Asp Leu Leu Phe Lys Asp Ser Ala His Gly Phe Leu Lys
    290             295             300

Val Pro Pro Arg Met Asp Ala Lys Met Tyr Leu Gly Tyr Glu Tyr Val
305             310             315             320

Thr Ala Ile Arg Asn Leu Arg Glu Gly Thr Cys Pro Glu Ala Pro Thr
            325             330             335

Asp Glu Cys Lys Pro Val Lys Trp Cys Ala Leu Ser His His Glu Arg
            340             345             350

Leu Lys Cys Asp Glu Trp Ser Val Asn Ser Val Gly Lys Ile Glu Cys
            355             360             365

```
Val Ser Ala Glu Thr Thr Glu Asp Cys Ile Ala Lys Ile Met Asn Gly
    370                 375             380

Glu Ala Asp Ala Met Ser Leu Asp Gly Gly Phe Val Tyr Ile Ala Gly
385             390                 395                     400

Lys Cys Gly Leu Val Pro Val Leu Ala Glu Asn Tyr Asn Lys Ser Asp
                405             410                 415

Asn Cys Glu Asp Thr Pro Glu Ala Gly Tyr Phe Ala Val Ala Val Val
            420             425             430

Lys Lys Ser Ala Ser Asp Leu Thr Trp Asp Asn Leu Lys Gly Lys Lys
        435             440                 445

Ser Cys His Thr Ala Val Gly Arg Thr Ala Gly Trp Asn Ile Pro Met
    450             455             460

Gly Leu Leu Tyr Asn Lys Ile Asn His Cys Arg Phe Asp Glu Phe Phe
465             470             475                     480

Ser Glu Gly Cys Ala Pro Gly Ser Lys Lys Asp Ser Ser Leu Cys Lys
            485             490                 495

Leu Cys Met Gly Ser Gly Leu Asn Leu Cys Glu Pro Asn Asn Lys Glu
        500             505                 510

Gly Tyr Tyr Gly Tyr Thr Gly Ala Phe Arg Cys Leu Val Glu Lys Gly
    515             520                 525

Asp Val Ala Phe Val Lys His Gln Thr Val Pro Gln Asn Thr Gly Gly
    530             535                 540

Lys Asn Pro Asp Pro Trp Ala Lys Asn Leu Asn Glu Lys Asp Tyr Glu
545             550                 555                     560

Leu Leu Cys Leu Asp Gly Thr Arg Lys Pro Val Glu Glu Tyr Ala Asn
            565             570                 575

Cys His Leu Ala Arg Ala Pro Asn His Ala Val Val Thr Arg Lys Asp
        580             585                 590

Lys Glu Ala Cys Val His Lys Ile Leu Arg Gln Gln Gln His Leu Phe
    595                 600                 605
```

27

```
Gly Ser Asn Val Thr Asp Cys Ser Gly Asn Phe Cys Leu Phe Arg Ser
    610             615             620

Glu Thr Lys Asp Leu Leu Phe Arg Asp Asp Thr Val Cys Leu Ala Lys
625             630             635             640

Leu His Asp Arg Asn Thr Tyr Glu Lys Tyr Leu Gly Glu Glu Tyr Val
                645             650             655

Lys Ala Val Gly Asn Leu Arg Lys Cys Ser Thr Ser Ser Leu Leu Glu
            660             665             670

Ala Cys Thr Phe Arg Arg Pro
            675
```

<210> 2
<211> 691
<212> PRT
<213> Homo sapiens

<220>
<221> MISC_FEATURE
<222> (1)..(691)
<223> Homo sapiens lactoferrin

<400> 2

```
Gly Arg Arg Arg Ser Val Gln Trp Cys Ala Val Ser Gln Pro Glu Ala
1               5               10              15

Thr Lys Cys Phe Gln Trp Gln Arg Asn Met Arg Lys Val Arg Gly Pro
            20              25              30

Pro Val Ser Cys Ile Lys Arg Asp Ser Pro Ile Gln Cys Ile Gln Ala
            35              40              45

Ile Ala Glu Asn Arg Ala Asp Ala Val Thr Leu Asp Gly Gly Phe Ile
    50              55              60

Tyr Glu Ala Gly Leu Ala Pro Tyr Lys Leu Arg Pro Val Ala Ala Glu
65              70              75              80

Val Tyr Gly Thr Glu Arg Gln Pro Arg Thr His Tyr Tyr Ala Val Ala
                85              90              95

Val Val Lys Lys Gly Gly Ser Phe Gln Leu Asn Glu Leu Gln Gly Leu
            100             105             110
```

```
Lys Ser Cys His Thr Gly Leu Arg Arg Thr Ala Gly Trp Asn Val Pro
        115                 120             125

Ile Gly Thr Leu Arg Pro Phe Leu Asn Trp Ala Gly Pro Pro Glu Pro
        130                 135             140

Ile Glu Ala Ala Val Ala Arg Phe Phe Ser Ala Ser Cys Val Pro Gly
145                 150             155               160

Ala Asp Lys Gly Gln Phe Pro Asn Leu Cys Arg Leu Cys Ala Gly Thr
                165             170             175

Gly Glu Asn Lys Cys Ala Phe Ser Ser Gln Glu Pro Tyr Phe Ser Tyr
            180             185             190

Ser Gly Ala Phe Lys Cys Leu Arg Asp Gly Ala Gly Asp Val Ala Phe
        195             200             205

Ile Arg Glu Ser Thr Val Phe Glu Asp Leu Ser Asp Glu Ala Glu Arg
    210             215             220

Asp Glu Tyr Glu Leu Leu Cys Pro Asp Asn Thr Arg Lys Pro Val Asp
225             230             235             240

Lys Phe Lys Asp Cys His Leu Ala Arg Val Pro Ser His Ala Val Val
            245             250             255

Ala Arg Ser Val Asn Gly Lys Glu Asp Ala Ile Trp Asn Leu Leu Arg
            260             265             270

Gln Ala Gln Glu Lys Phe Gly Lys Asp Lys Ser Pro Lys Phe Gln Leu
        275             280             285

Phe Gly Ser Pro Ser Gly Gln Lys Asp Leu Leu Phe Lys Asp Ser Ala
    290             295             300

Ile Gly Phe Ser Arg Val Pro Pro Arg Ile Asp Ser Gly Leu Tyr Leu
305             310             315             320

Gly Ser Gly Tyr Phe Thr Ala Ile Gln Asn Leu Arg Lys Ser Glu Glu
            325             330             335

Glu Val Ala Ala Arg Arg Ala Arg Val Val Trp Cys Ala Val Gly Glu
        340             345             350
```

```
Gln Glu Leu Arg Lys Cys Asn Gln Trp Ser Gly Leu Ser Glu Gly Ser
        355             360             365

Val Thr Cys Ser Ser Ala Ser Thr Thr Glu Asp Cys Ile Ala Leu Val
        370             375             380

Leu Lys Gly Glu Ala Asp Ala Met Ser Leu Asp Gly Gly Tyr Val Tyr
385             390             395             400

Thr Ala Gly Lys Cys Gly Leu Val Pro Val Leu Ala Glu Asn Tyr Lys
            405             410             415

Ser Gln Gln Ser Ser Asp Pro Asp Pro Asn Cys Val Asp Arg Pro Val
            420             425             430

Glu Gly Tyr Leu Ala Val Ala Val Val Arg Arg Ser Asp Thr Ser Leu
            435             440             445

Thr Trp Asn Ser Val Lys Gly Lys Lys Ser Cys His Thr Ala Val Asp
    450             455             460

Arg Thr Ala Gly Trp Asn Ile Pro Met Gly Leu Leu Phe Asn Gln Ala
465             470             475             480

Gly Ser Cys Lys Phe Asp Glu Tyr Phe Ser Gln Ser Cys Ala Pro Gly
            485             490             495

Ser Asp Pro Arg Ser Asn Leu Cys Ala Leu Cys Ile Gly Asp Glu Gln
            500             505             510

Gly Glu Asn Lys Cys Val Pro Asn Ser Asn Glu Arg Tyr Tyr Gly Tyr
            515             520             525

Thr Gly Ala Phe Arg Cys Leu Ala Glu Asn Ala Gly Asp Val Ala Phe
    530             535             540

Val Lys Asp Val Thr Val Leu Gln Asn Thr Asp Gly Asn Asn Asn Glu
545             550             555             560

Ala Trp Ala Lys Asp Leu Lys Leu Ala Asp Phe Ala Leu Leu Cys Leu
            565             570             575

Asp Gly Lys Arg Lys Pro Val Thr Glu Ala Arg Ser Cys His Leu Ala
            580             585             590
```

```
Met Ala Pro Asn His Ala Val Val Ser Arg Met Asp Lys Val Glu Arg
        595             600             605

Leu Lys Gln Val Leu Leu His Gln Gln Ala Lys Phe Gly Arg Asn Gly
    610             615             620

Ala Asp Cys Pro Asp Lys Phe Cys Leu Phe Gln Ser Glu Thr Lys Asn
625             630             635             640

Leu Leu Phe Asn Asp Asn Thr Glu Cys Leu Ala Arg Leu His Gly Lys
            645             650             655

Thr Thr Tyr Glu Lys Tyr Leu Gly Pro Gln Tyr Val Ala Gly Ile Thr
        660             665             670

Asn Leu Lys Lys Cys Ser Thr Ser Pro Leu Leu Glu Ala Cys Glu Phe
        675             680             685

Leu Arg Lys
    690
```

<210> 3
<211> 738
<212> PRT
<213> Homo sapiens

<220>
<221> MISC_FEATURE
<222> (1)..(738)
<223> Homo sapiens lactoferrin

<400> 3

```
Met Arg Gly Pro Ser Gly Ala Leu Trp Leu Leu Leu Ala Leu Arg Thr
1               5                   10                  15

Val Leu Gly Gly Met Glu Val Arg Trp Cys Ala Thr Ser Asp Pro Glu
            20                  25                  30

Gln His Lys Cys Gly Asn Met Ser Glu Ala Phe Arg Glu Ala Gly Ile
        35                  40                  45

Gln Pro Ser Leu Leu Cys Val Arg Gly Thr Ser Ala Asp His Cys Val
    50                  55                  60

Gln Leu Ile Ala Ala Gln Glu Ala Asp Ala Ile Thr Leu Asp Gly Gly
                    70                  75                  80
```

```
Ala Ile Tyr Glu Ala Gly Lys Glu His Gly Leu Lys Pro Val Val Gly
                85                  90              95

Glu Val Tyr Asp Gln Glu Val Gly Thr Ser Tyr Tyr Ala Val Ala Val
                100             105             110

Val Arg Arg Ser Ser His Val Thr Ile Asp Thr Leu Lys Gly Val Lys .
            115             120             125

Ser Cys His Thr Gly Ile Asn Arg Thr Val Gly Trp Asn Val Pro Val
    130             135             140

Gly Tyr Leu Val Glu Ser Gly Arg Leu Ser Val Met Gly Cys Asp Val
145             150             155             160

Leu Lys Ala Val Ser Asp Tyr Phe Gly Gly Ser Cys Val Pro Gly Ala
                165             170             175

Gly Glu Thr Ser Tyr Ser Glu Ser Leu Cys Arg Leu Cys Arg Gly Asp
            180             185             190

Ser Ser Gly Glu Gly Val Cys Asp Lys Ser Pro Leu Glu Arg Tyr Tyr
    .       195             200             205

Asp Tyr Ser Gly Ala Phe Arg Cys Leu Ala Glu Gly Ala Gly Asp Val
    210             215             220

Ala Phe Val Lys His Ser Thr Val Leu Glu Asn Thr Asp Gly Lys Thr
225             230             235             240

Leu Pro Ser Trp Gly Gln Ala Leu Leu Ser Gln Asp Phe Glu Leu Leu
            245             250             255

Cys Arg Asp Gly Ser Arg Ala Asp Val Thr Glu Trp Arg Gln Cys His
            260             265             270

Leu Ala Arg Val Pro Ala His Ala Val Val Val Arg Ala Asp Thr Asp
    275             280             285

Gly Gly Leu Ile Phe Arg Leu Leu Asn Glu Gly Gln Arg Leu Phe Ser
    290             295             300

His Glu Gly Ser Ser Phe Gln Met Phe Ser Ser Glu Ala Tyr Gly Gln
    5               310             315             320
```

Lys Asp Leu Leu Phe Lys Asp Ser Thr Ser Glu Leu Val Pro Ile Ala
                325                     330                     335

Thr Gln Thr Tyr Glu Ala Trp Leu Gly His Glu Tyr Leu His Ala Met
                340                     345                     350

Lys Gly Leu Leu Cys Asp Pro Asn Arg Leu Pro Pro Tyr Leu Arg Trp
                355                     360                     365

Cys Val Leu Ser Thr Pro Glu Ile Gln Lys Cys Gly Asp Met Ala Val
        370                     375                     380

Ala Phe Arg Arg Gln Arg Leu Lys Pro Glu Ile Gln Cys Val Ser Ala
385                     390                     395                     400

Lys Ser Pro Gln His Cys Met Glu Arg Ile Gln Ala Glu Gln Val Asp
                405                     410                     415

Ala Val Thr Leu Ser Gly Glu Asp Ile Tyr Thr Ala Gly Lys Thr Tyr
                420                     425                     430

Gly Leu Val Pro Ala Ala Gly Glu His Tyr Ala Pro Glu Asp Ser Ser
                435                     440                     445

Asn Ser Tyr Tyr Val Val Ala Val Val Arg Arg Asp Ser Ser His Ala
        450                     455                     460

Phe Thr Leu Asp Glu Leu Arg Gly Lys Arg Ser Cys His Ala Gly Phe
465                     470                     475                     480

Gly Ser Pro Ala Gly Trp Asp Val Pro Val Gly Ala Leu Ile Gln Arg
                485                     490                     495

Gly Phe Ile Arg Pro Lys Asp Cys Asp Val Leu Thr Ala Val Ser Glu
                500                     505                     510

Phe Phe Asn Ala Ser Cys Val Pro Val Asn Asn Pro Lys Asn Tyr Pro
        515                     520                     525

Ser Ser Leu Cys Ala Leu Cys Val Gly Asp Glu Gln Gly Arg Asn Lys
        530                     535                     540

Cys Val Gly Asn Ser Gln Glu Arg Tyr Tyr Gly Tyr Arg Gly Ala Phe
    5               550                     555                     560

34

Arg Cys Leu Val Glu Asn Ala Gly Asp Val Ala Phe Val Arg His Thr
                    565                 570             575

Thr Val Phe Asp Asn Thr Asn Gly His Asn Ser Glu Pro Trp Ala Ala
                580                 585             590

Glu Leu Arg Ser Glu Asp Tyr Glu Leu Leu Cys Pro Asn Gly Ala Arg
            595             600             605

Ala Glu Val Ser Gln Phe Ala Ala Cys Asn Leu Ala Gln Ile Pro Pro
    610             615             620

His Ala Val Met Val Arg Pro Asp Thr Asn Ile Phe Thr Val Tyr Gly
625             630             635             640

Leu Leu Asp Lys Ala Gln Asp Leu Phe Gly Asp Asp His Asn Lys Asn
            645             650             655

Gly Phe Lys Met Phe Asp Ser Ser Asn Tyr His Gly Gln Asp Leu Leu
            660             665             670

Phe Lys Asp Ala Thr Val Arg Ala Val Pro Val Gly Glu Lys Thr Thr
            675             680             685

Tyr Arg Gly Trp Leu Gly Leu Asp Tyr Val Ala Ala Leu Glu Gly Met
    690             695             700

Ser Ser Gln Gln Cys Ser Gly Ala Ala Ala Pro Ala Pro Gly Ala Pro
705             710             715             720

Leu Leu Pro Leu Leu Leu Pro Ala Leu Ala Ala Arg Leu Leu Pro Pro
            725             730             735

Ala Leu

<210> 4
<211> 679
<212> PRT
<213> Artificial sequence

<220>
<223> Artificial variant of Homo sapiens transferrin

<400> 4

Val Pro Asp Lys Thr Val Arg Trp Cys Ala Val Ser Glu His Glu Ala
1           5               10              15

Thr Lys Cys Gln Ser Phe Arg Asp His Met Lys Ser Val Ile Pro Ser
        20              25              30

Asp Gly Pro Ser Val Ala Cys Val Lys Lys Ala Ser Tyr Leu Asp Cys
        35              40              45

Ile Arg Ala Ile Ala Ala Asn Glu Ala Asp Ala Val Thr Leu Asp Ala
    50              55              60

Gly Leu Val Tyr Asp Ala Tyr Leu Ala Pro Asn Asn Leu Lys Pro Val
65              70              75              80

Val Ala Glu Phe Tyr Gly Ser Lys Glu Asp Pro Gln Thr Phe Tyr Tyr
            85              90              95

Ala Val Ala Val Val Lys Lys Asp Ser Gly Phe Gln Met Asn Gln Leu
        100             105             110

Arg Gly Lys Lys Ser Cys His Thr Gly Leu Gly Arg Ser Ala Gly Trp
        115             120             125

Asn Ile Pro Ile Gly Leu Leu Tyr Cys Asp Leu Pro Glu Pro Arg Lys
    130             135             140

Pro Leu Glu Lys Ala Val Ala Asn Phe Phe Ser Gly Ser Cys Ala Pro
145             150             155             160

Cys Ala Asp Gly Thr Asp Phe Pro Gln Leu Cys Gln Leu Cys Pro Gly
            165             170             175

Cys Gly Cys Ser Thr Leu Asn Gln Tyr Phe Gly Tyr Ser Gly Ala Phe
            180             185             190

Lys Cys Leu Lys Asp Gly Ala Gly Asp Val Ala Phe Val Lys His Ser
    195             200             205

Thr Ile Phe Glu Asn Leu Ala Asn Lys Ala Asp Arg Asp Gln Tyr Glu
    210             215             220

Leu Leu Cys Leu Asp Asn Thr Arg Lys Pro Val Asp Glu Tyr Lys Asp
225             230             235             240

```
Cys His Leu Ala Gln Val Pro Ser His Thr Val Val Ala Arg Ser Met
                245             250                     255


Gly Gly Lys Glu Asp Leu Ile Trp Glu Leu Leu Asn Gln Ala Gln Glu
            260             265                 270


His Phe Gly Lys Asp Lys Ser Lys Glu Phe Gln Leu Phe Ser Ser Pro
            275             280                 285


His Gly Lys Asp Leu Leu Phe Lys Asp Ser Ala His Gly Phe Leu Lys
        290             295                 300


Val Pro Pro Arg Met Asp Ala Lys Met Tyr Leu Gly Tyr Glu Tyr Val
305                 310                 315                 320


Thr Ala Ile Arg Asn Leu Arg Glu Gly Thr Cys Pro Glu Ala Pro Thr
                325             330                 335


Asp Glu Cys Lys Pro Val Lys Trp Cys Ala Leu Ser His His Glu Arg
            340             345                 350


Leu Lys Cys Asp Glu Trp Ser Val Asn Ser Val Gly Lys Ile Glu Cys
            355             360                 365


Val Ser Ala Glu Thr Thr Glu Asp Cys Ile Ala Lys Ile Met Asn Gly
    370                 375                 380


Glu Ala Asp Ala Met Ser Leu Asp Gly Gly Phe Val Tyr Ile Ala Gly
385                 390                 395                 400


Lys Cys Gly Leu Val Pro Val Leu Ala Glu Asn Tyr Asn Lys Ala Asp
            405                 410                 415


Asn Cys Glu Asp Thr Pro Glu Ala Gly Tyr Phe Ala Val Ala Val Val
            420             425                 430


Lys Lys Ser Ala Ser Asp Leu Thr Trp Asp Asn Leu Lys Gly Lys Lys
        435             440                 445


Ser Cys His Thr Ala Val Gly Arg Thr Ala Gly Trp Asn Ile Pro Met
    450                 455                 460


Gly Leu Leu Tyr Asn Lys Ile Asn His Cys Arg Phe Asp Glu Phe Phe
465             470                 475                 480
```

Ser Glu Gly Cys Ala Pro Gly Ser Lys Lys Asp Ser Ser Leu Cys Lys
485                     490                         495

Leu Cys Met Gly Ser Gly Leu Asn Leu Cys Glu Pro Asn Asn Lys Glu
500                     505                     510

Gly Tyr Tyr Gly Tyr Thr Gly Ala Phe Arg Cys Leu Val Glu Lys Gly
515                     520                     525

Asp Val Ala Phe Val Lys His Gln Thr Val Pro Gln Asn Thr Gly Gly
530                     535                     540

Lys Asn Pro Asp Pro Trp Ala Lys Asn Leu Asn Glu Lys Asp Tyr Glu
545                     550                     555                     560

Leu Leu Cys Leu Asp Gly Thr Arg Lys Pro Val Glu Glu Tyr Ala Asn
565                     570                     575

Cys His Leu Ala Arg Ala Pro Asn His Ala Val Val Thr Arg Lys Asp
580                     585                     590

Lys Glu Ala Cys Val His Lys Ile Leu Arg Gln Gln Gln His Leu Phe
595                     600                     605

Gly Ser Asn Val Ala Asp Cys Ser Gly Asn Phe Cys Leu Phe Arg Ser
610                     615                     620

Glu Thr Lys Asp Leu Leu Phe Arg Asp Asp Thr Val Cys Leu Ala Lys
625                     630                     635                     640

Leu His Asp Arg Asn Thr Tyr Glu Lys Tyr Leu Gly Glu Glu Tyr Val
645                     650                     655

Lys Ala Val Gly Asn Leu Arg Lys Cys Ser Thr Ser Ser Leu Leu Glu
660                     665                     670

Ala Cys Thr Phe Arg Arg Pro
675